# EUROPÄISCHE PATENTSCHRIFT

(11) **EP 2 054 093 B1**
(45) Veröffentlichungstag und Bekanntmachung des Hinweises auf die Patenterteilung: **21.11.2012**
(21) Anmeldenummer: 07785097.2
(22) Anmeldetag: 17.08.2007
(51) Int. Cl.: A61L 27/04, A61L 27/06, A61C 13/08

(54) **IMPLANTAT, INSBESONDERE DENTALIMPLANTAT**
IMPLANT, IN PARTICULAR DENTAL IMPLANT
IMPLANT, NOTAMMENT IMPLANT DENTAIRE

(30) Priorität: 22.08.2006 CH 13392006
(43) Veröffentlichungstag der Anmeldung: 06.05.2009
(60) Teilanmeldung: 12164089.0
(73) Patentinhaber: THOMMEN MEDICAL AG, 4437 Waldenburg (CH)
(72) Erfinder: SCHLOTTIG, Falko, CH-4414 Füllinsdorf (CH); SNETIVY, Daniel, CH-4147 Aesch (CH)
(74) Vertreter: Bremi, Tobias Hans
(86) Internationale Anmeldenummer: PCT/CH2007/000402
(87) Internationale Veröffentlichungsnummer: WO 2008/022478

(56) Entgegenhaltungen:
- JP-A- 2000 060 958
- DATABASE WPI Week 200358 Derwent Publications Ltd., London, GB; AN 2003-608626 XP002413203 & CN 1 417 378 A (UNIV TIANJIN) 14. Mai 2003 (2003-05-14)
- NISHIGUCHI SHIGERU ET AL: "Biology of alkali- and heat-treated titanium implants" J. BIOMED. MATER. RES. PART A; JOURNAL OF BIOMEDICAL MATERIALS RESEARCH - PART A OCT 1 2003, Bd. 67, Nr. 1, 1. Oktober 2003 (2003-10-01), Seiten 26-35, XP002413201

## Beschreibung

### TECHNISCHES GEBIET

Die Erfindung betrifft ein metallisches Implantat, insbesondere ein Dentalimplantat, mit einer hydrophilen Oberfläche zum wenigstens teilweisen Einsetzen in einen Knochen, sowie ein Verfahren zu dessen Herstellung.

### HINTERGRUND DER ERFINDUNG

Verletzte oder beschädigte Teile des Hart- und/oder Weichgewebes des menschlichen Körpers werden am Besten wiederhergestellt, indem körpereigenes Hart- und/oder Weichgewebe verwendet wird. Dies ist aus verschiedenen Gründen nicht immer möglich, und daher kommt in vielen Fällen synthetisches Material als temporäres (bioabbaubares respektive postoperativ entfernbares) oder permanentes Ersatzmaterial zum Einsatz.

Implantate, welche im Hart- und/oder Weichgewebe verankert werden, dienen dem temporären oder permanenten Ersatz oder dem Support von unfall-, abnützungs-, mangelerscheinungs- oder krankheitsgeschädigten oder sonst degenerierten Teilen des Bewegungsapparates, einschliesslich insbesondere des Kauapparates. Als Implantat wird normalerweise ein künstliches, chemisch stabiles Material bezeichnet, welches als plastischer Ersatz oder zur mechanischen Verstärkung in den Körper eingebracht wird (vgl. z.B. Roche Lexikon Medizin, Urban & Fischer, (Hrsg.); 5. Aufl. 2003). Die Hilfs- und Ersatzfunktion im Körper wird auf Basis der mechanischen Eigenschaften und des Implantatdesigns übernommen. So sind beispielsweise Hüft- und Kniegelenksprothesen, Wirbelsäulenimplantate und Dentalimplantate seit vielen Jahren im erfolgreichen klinischen Einsatz.

Für die Implantatverankerung und die Implantatverträglichkeit an der Grenzfläche Implantatoberfläche / angrenzendes Gewebe hat die Implantatoberfläche eine grosse Bedeutung. So haben Messungen gezeigt, dass Implantate mit glatter Oberfläche beinahe unabhängig vom verwendeten Basismaterial nur wenig im Knochen verankert werden (schlechte Osteointegration), während Implantate mit einer strukturierten Oberfläche eine gute mechanische und bei entsprechender Gestaltung der Oberfläche auch eine gute biologische Verbindung mit dem umgebenden Hart- oder Weichgewebe eingehen (vgl. z.B. Titanium in Medicine, Material Science, Surface Science, Engineering, biological Responses and Medical Applications Series: Engineering Materials, Brunette, D.M.; Tengvall, P.; Textor, M.; Thomsen, P. (Eds.)).

Die für ein genügendes Einwachsen benötigte Zeit, eine für Implantate wichtige und zentrale Eigenschaft, wird als Osteointegrationszeit, respektive im Dentalbereich auch als Osseointegrationszeit bezeichnet. Damit wird die Zeit beschrieben, die vergeht, bis sich die Knochensubstanz in genügender Stärke und dauerhaft mit der Implantatoberfläche verbunden hat, das heisst sich gewissermassen in diese integriert hat.

Zur Oberflächenbehandlung werden verschiedenste Methoden verwendet, siehe z.B. A Guide to Metal and Plastic Finishing (Maroney, Marion L.; 1991); Handbook of Semiconductor Electrodeposition (Applied Physics, 5) (Pandey, R. K., et. al.; 1996); Surface Finishing Systems: Metal and Non-Metal Finishing Handbook-Guide (Rudzki, George J.; 1984); Titanium in Medicine, Material Science, Surface Science, Engineering, Biological Responses and Medical Applications Series: Engineering Materials, (Brunette, D.M.; Tengvall, P.; Textor, M.; Thomsen, P. (Eds.)) und Materials and Processes for Surface and Interface Engineering (NATO Asi Series. Series E, Applied Sciences, 115, Pauleau, Ives (Editor); 1995); und die darin genannten Referenzen.

Neben der Oberflächentopographie kann die Osseointegration des Implantates durch chemische Beschichtungen oder Modifizierungen der Oberfläche beeinflusst werden. So können Implantate in einer wässrigen Calcium- und Phosphationen-haltigen Lösung beschichtet werden. Die resultierende Oberfläche besteht aus den beiden Calciumphosphat-Phasen Hydroxylapatit und Bruschit. Diese Beschichtung wird direkt auf der Implantatoberfläche innerhalb von 6 - 10 Wochen durch jungen Knochen postoperativ ersetzt und führt zu einem sehr guten Einheilen der Implantate (Zeggel P, Bioactive Calcium Phosphate Coatings for Dental Implants, International Magazine Of Oral Implantology, 1/2000).

Die direkte Modifizierung einer optimierten rauen Oberfläche mit Fluorid auf Titanimplantaten wird von Ellingsen (Ellingsen, J. E. et al. Improved Retention and Bone to Implant Contact with Fluoride Modified Titanium Implants Int. J. Oral Maxillofac Implants (2004); Vol. 19; S. 659-666) als vorteilhaft für den Knocheneinheilprozesses beschrieben.

Eine chemisch aktive, hydrophile Implantatoberfläche auf Titanimplantaten kann über einen sehr aufwendigen Konservierungsprozess in Stickstoffatmosphäre hergestellt werden. Die Lagerung der Oberfläche in einer Lösung aus Natriumchlorid konserviert die hydrophilen Eigenschaften. Eine solche Oberfläche soll den Prozess der Osseointegration beschleunigen und zu einer höheren Implantatstabilität in der frühen Phase der Osseointegration führen (Ferguson S.J. et al, Biomechanical evaluation of the interfacial strength of a chemically modified sandblasted and acid-etched titanium surface, Journal of Biomedical Materials Research Part A Volume 78A, Issue 2, Pages 291 - 297). Tierstudien der hydrophilen Oberfläche zeigen einen signifikant höheren Knochen-Implantat Kontakt im Vergleich mit einer hydrophoben Oberfläche bei gleicher Oberflächentopopgraphie (Buser D. et al, Enhanced Bone Apposition to a Chemically Modified SLA Titanium Surface, J. Dent. Res. 83 (7) 529-533 (2004).). Diese beschriebenen hydrophilen Eigenschaften können technologisch nur sehr aufwendig hergestellt und durch eine spezielle Lagerung erhalten werden, bei längerem Luftkontakt geht die Oberfläche in den hydrophoben Zustand über. Problematisch für diese Technologie sind weiterhin die hohen Herstellungs- und Verpackungskosten und die begrenzte Lagerdauer in einer salinen Lösung

Aus der JP 2000-060958 ist ein Verfahren bekannt, bei welchem ein Implantat zunächst in einem ersten Schritt einer hoch konzentrierten Natriumhydroxid Lösung mit einer Konzentration von 5 mol/l behandelt wird, dann unter Einfluss von Hitze gesintert wird, und in einem zweiten Schritt mit einer Calciumhydroxid Lösung mit einer Konzentration im Bereich von 0.1-20 mol/l Während einer Dauer von 10 Minuten bis drei Tagen bei erhöhter Temperatur von mehr als 50°C behandelt und anschliessend ausdrücklich gewaschen wird. Dabei bildet sich an der Oberfläche angeblich Apatit, was für das Einwachsen des Implantats vorteilhafte Wirkungen zeigen soll.

### ALLGEMEINE DARSTELLUNG DER ERFINDUNG

Der Erfindung liegt somit die Aufgabe zugrunde, die Nachteile des Standes der Technik zu vermeiden und Implantate vorzuschlagen, welche eine hydrophile Oberfläche besitzen und welche im Hart- und Weichgewebe zügig und nachhaltig verankern und somit gute Osteointegration resp. Osseointegration zeigen. Konkret geht es also darum, ein verbessertes Implantat mit einer bevorzugt strukturierten, und chemisch modifizierten Oberfläche zum wenigstens teilweisen Einsetzen in Hartgewebe wie in einen Knochen und/oder in Weichgewebe vorzuschlagen, wobei das Implantat metallisch ist. Ferner soll ein geeignetes Herstellverfahren hierfür angegeben werden. Die Lösung dieser Aufgabe wird dadurch erreicht, dass die wenigstens bereichsweise chemisch modifizierte und damit hydrophile Oberfläche das Resultat einer basischen Oberflächenbehandlung ist. Diese Aufgabe wird erfindungsgemäss also durch eine spezifisch behandelte und dadurch spezifische Eigenschaften aufweisende Oberfläche des Implantates gelöst, wobei die Behandlung sowohl über die gesamte Implantatoberfläche als auch von partiellen Anteilen der Implantatoberfläche erfolgen kann. Im Rahmen dieser Erfindung ist vorliegend zunächst von Implantaten die Rede, welche auf metallischen Materialien beruhen.

Die Begriffe hydrophil und hydrophob beschreiben die Benetzbarkeit einer Oberfläche. Dabei wird eine Oberfläche als hydrophil beschrieben, wenn sie benetzbar ist, der Fall des Nichtbenetzens wird als hydrophob bezeichnet. Die hydrophilen oder hydrophoben Eigenschaften können quantitativ über Kontaktwinkelmessungen ermittelt werden. Als Kontaktwinkel wird dabei der Winkel bezeichnet, den ein Flüssigkeitstropfen auf der Oberfläche eines Feststoffes zu dieser Oberfläche bildet. Bei der Verwendung von Wasser als Flüssigkeit bezeichnet man bei Kontaktwinkeln unter 90° die Oberfläche als hydrophil, bei grössser als 90° als hydrophob. Implantate mit einer eher hydrophilen Oberfläche zeigen eine bessere und schnellere Osseointegration (Ferguson SJ, Broggini N, Wieland M, de Wild M, Rupp F, Geis-Gerstorfer J, Cochran DL, Buser D.: Biomechanical evaluation of the interfacial strength of a chemically modified sandblasted and acid-etched titanium surface; J Biomed Mater Res A. 2006 Aug;78(2):291-7 sowie Rupp F, Scheideler L, Olshanska N, de Wild M, Wieland M, Geis-Gerstorfer J.: Enhancing surface free energy and hydrophilicity through chemical modification of microstructured titanium implant surfaces; J Biomed Mater Res A. 2006 Feb; 76(2):323-34.

Der Kern der Erfindung besteht somit darin, dass überraschenderweise festgestellt wurde, dass insbesondere Implantate auf Basis von Metall unter Verwendung einer spezifischen basischen Behandlung an der Oberfläche derart modifiziert werden können, dass sie anschliessend hervorragende Osteointegration resp. Osseointegration zeigen. Es zeigt sich, dass die Osteointegration resp. Osseointegration einer derart hydrophilisierten Oberfläche besser ist, als die entsprechenden Werte für nur gestrahlte und säuregeätzte Oberflächen und/oder Oberflächen insbesondere von Metallen, welche nur durch Sandstrahlen und Ätzen mit einer Makro- und Mikrorauhigkeit versehen wurden.

Das Implantat wird also an der Oberfläche durch eine basische Behandlung modifiziert, wobei insbesondere bevorzugt bei der Behandlung in basischer Lösung im wesentlichen ausschliesslich eine Hydrophilisierung der Oberfläche erfolgt. Die Behandlung in der basischen Lösung erfolgt insbesondere ohne Anlegen eines elektrischen Potenzials, das heisst das Implantat wird ganz einfach in die Lösung eingetaucht. Die Oberflächenbehandlung führt zu einer hydrophilen Oberfläche, die ohne zusätzliche aufwendige Lagerung eine bestimmte Zeit hydrophil bleibt. Es geht mit anderen Worten nicht darum, durch die basische Behandlung nur beispielsweise Anionen oder Kationen aus der basischen Lösung in das Grundmaterial einzubringen oder gewissermassen eine Beschichtung oder einen topographischen Abtrag zu bewirken, sondern es geht effektiv darum, durch die basische Lösung einen die Hydrophilie der Oberfläche verändernden chemischen Prozess zu benutzen, um eine spezifisch hydrophile Oberfläche zu erhalten.

Tatsächlich wird auch gefunden, dass die erfindungsgemäss hergestellten Oberflächen, im Gegensatz zu Behandlungen nach dem Stand der Technik mit stark basischen Lösungen (normalerweise im Bereich von 5M - 20M Hydroxid-Lösungen), im wesentlichen keine topologischen resp. topographischen Strukturänderungen, welche auf die basische Behandlung zurückzuführen sind, aufweisen.

Die Hydrophilisierung der Oberfläche erfolgt bevorzugtermassen vollständig in basischer Umgebung. Die basische Umgebung können wässrige oder organische basische Lösungen sein. Die Oberflächenbehandlung kann gegebenenfalls mit einer mechanischen und chemisch abtragenden Behandlung zur Erzeugung der Topographie gekoppelt sein.

Zusätzliche respektive nachfolgende Beschichtungen wie beispielsweise aus Apatit sind nicht erforderlich und bevorzugtermassen auch nicht vorhanden.

Ebenfalls im Gegensatz zum Stand der Technik sind Nachbehandlungen mit hoher Temperatur (z.B. Behandlung bei 600°C während mehreren Stunden) oder ein aufwändiges Spülen (z.B. im Ultraschallbad) nicht erforderlich. Dies ist insbesondere dann von enormem Vorteil bei kalt verformten Werkstoffen wie z.B. Titan, welche sonst ihre mechanischen Eigenschaften verlieren würden.

Bevorzugtermassen handelt es sich dabei es sich um eine wässrige oder organische Lösung von einem oder mehreren Alkali-Hydroxiden (insb. NaOH), handelt, wobei bevorzugtermassen eine Gesamtkonzentration im Bereich von Bereich von 0.05M - 0.1M verwendet wird.

Als besonders geeignet erweist sich die hydrophile respektiv basisch modifizierte Oberfläche, wenn das Implantat wenigstens an der Oberfläche mit einer Schicht aus Metall respektive Metalloxid versehen ist, oder wenn, wie bevorzugt, das Implantat im wesentlichen vollständig aus Metall besteht. In diesem Zusammenhang muss herausgestrichen werden, dass die Oberfläche eines Implantats aus Titan, Zirkon, Hafnium, Tantal, Niob etc. sowie aus daraus gebildeten Legierungen (vgl. auch unten) beim Kontakt mit Sauerstoff, das heisst zum Beispiel an der Luft, sofort eine dünne oberflächliche Schicht aus dem entsprechenden Oxid ausbildet. Titan-Implantate verfügen beispielsweise über eine derartige dünne Schicht aus Titan (IV)-Dioxid (TiO₂) mit geringen Bestandteilen von Ti₂O₃ und TiO. Wenn in der Folge von metallischen Implantaten respektive deren Oberfläche gesprochen wird, so soll dies entsprechend eine Oberfläche mit einer solchen Oxidschicht mit einschliessen. Bei dem Metall kann es sich um unterschiedliche Typen handeln, wobei diese aus dem Stand der Technik bekannt sind. Beispielsweise kann ein Metall verwendet werden, welches aus reinem Titan nach ISO 5832 besteht. Alternativ ist es möglich, Metalle zu verwenden, welche als Implantatstahl nach ISO 5832 bekannt sind. Ebenfalls ist es möglich, Titanlegierungen zu verwenden, welche neben Titan Aluminium und/oder Vanadium und/oder Niob enthalten. Auch Metalle basierend auf Cobalt Chrom Legierungen, auf Cobalt Chrom Molybdän Legierungen, auf Cobalt Chrom Wolfram Nickel Legierungen und auf Cobalt Nickel Chrom Molybdän Titan Legierungen sind möglich. Ebenfalls sind Metalle wie Tantal oder Magnesium oder auf Tantal oder Magnesium basierende Legierungen möglich.

Gemäss einer bevorzugten Ausführungsform handelt es sich beim Implantat um ein Dentalimplantat, dessen im implantierten Zustand dem Knochen und/oder Weichgewebe ausgesetzte Oberfläche wenigstens bereichsweise hydrophilisiert ist. Die hydrophilisierte Oberfläche kann auf einer topograpisch vorstrukturierten Oberfläche erzeugt werden. Es kann sich dabei um eine sandstrahl- und/oder ätzmodifizierte Oberfläche handeln. Des weiteren betrifft die vorliegende Erfindung ein Verfahren zur Herstellung eines Implantates, wie es oben beschrieben wird. Das Verfahren ist dadurch gekennzeichnet, dass ein Implantat aus Metall gegebenenfalls nach vorgängiger abtragender Oberflächenmodifikation insbesondere zur Erzeugung einer Makro und Mikrorauhigkeit (z.B. auch in einer Salzschmelze), wenigstens in den Knochen und/oder Weichgewebe ausgesetzten Bereichen unter Zuhilfenahme einer basischen Oberflächebehandlung oberflächenmodifiziert wird.

Spezifisch ist das Verfahren zur Herstellung eines metallischen Implantats mit einer hydrophilen Oberfläche zum wenigstens teilweisen Einsetzen in Hartgewebe wie in einen Knochen und/oder in Weichgewebe, dadurch gekennzeichnet, dass das Verfahren wenigstens einen Schritt umfasst, in welchem wenigstens ein zum teilweisen Einsetzen in Hartgewebe wie in einen Knochen und/oder in Weichgewebe vorgesehener Bereich, gegebenenfalls nach vorgängiger mechanisch und/oder chemisch insbesondere abtragender Oberflächenmodifikation, in einer wässrigen oder organischen Lösung von Alkali -Hydroxiden, oder einer Mischung dieser Hydroxide, mit einer Gesamtkonzentration von Alkali- -Hydroxid im Bereich von 0.005M - 0.1M kurzzeitig behandelt wird. Bevorzugt werden Konzentrationen in diesem Bereich, die Untergrenze kann aber auch bei 0.01 M liegen. Bezüglich der Obergrenze kann diese auch bevorzugt bei 0.07 M oder 0.05 M liegen. Die genannten Untergrenzen und Obergrenzen können entsprechend kombiniert werden.

Nach der Erfindung werden in dem Schritt der Alkali-Hydroxid-Behandlung Metalloxide an der Oberfläche modifiziert.

Nach der Erfindung wird eine basische Lösung im wesentlichen aus Alkalihydroxiden, wie z.B. aus Kaliumhydroxid und/oder Natriumhydroxid verwendet. Geringe Bestandteile, typischerweise im Bereich von weniger als 5% oder sogar weniger als 2%, anderer Salze (nicht nur, aber bevorzugtermassen der oben genannten) oder anderer Additive, zur Einstellung der Hydrophilisierungsbedingungen können zusätzlich vorhanden sein.

Bevorzugtermassen handelt es sich bei der basischen Lösung um eine wässrige Lösung ausschliesslich bestehend aus einem oder mehreren der genannten Hydroxide.

Eine weitere bevorzugte Ausführungsform ist dadurch gekennzeichnet, dass in einer wässrigen Lösung von Alkali- und/oder Erdalkali-Hydroxiden, oder einer Mischung dieser Hydroxide, mit einer Gesamtkonzentration von Alkali- und/oder Erdalkali-Hydroxid im Bereich von 0.05M - 0.1M kurzzeitig behandelt wird. Generell ist nach der Erfindung, dass die Behandlung während einer vergleichsweise kurzen Zeitspanne z.B. im Bereich von 2sec - 10 min, bevorzugt von 5sec - 120 sec, insbesondere bevorzugt von 5 - 30 sec stattfindet.

Weiterhin ist nach der Erfindung, die Behandlung in einer wässrigen Lösung von Alkali- -Hydroxiden, oder einer Mischung dieser Hydroxide, bei einer Temperatur im Bereich von 10 - 30° stattfinden zu lassen.

Eine weitere bevorzugte Ausführungsform ist dadurch gekennzeichnet, dass es sich bei der wässrigen Lösung um eine Lösung aus Natriumhydroxid handelt. Dies bevorzugt in einer Konzentration von 0.01 - 0.1 Mol bevorzugt von 0.01 - 0.07 Mol, wobei bei einer Temperatur im Bereich von im Bereich von 10 - 30 °C (z.B. RT) gearbeitet wird.

Wie bereits erläutert, ist das Verfahren dadurch gekennzeichnet, dass die Implantatoberfläche nach der Behandlung mit der wässrigen Lösung ohne Nachbehandlung mit erhöhter Temperatur und/oder Nachbehandlung durch Spülen gelagert und/oder verpackt und/oder implantiert werden kann.

Eine weitere Ausführungsform betrifft ein Lagerungsverfahren, welches dadurch gekennzeichnet ist, dass die Implantatoberfläche nach der Behandlung mit der wässrigen Lösung in einer basischen Lösung gelagert und verpackt wird. Bei der basischen Lösung kann es sich um eine Lösung von bevorzugt ausschliesslich Alkali- Hydroxiden, oder eine Mischung dieser Lösungen handeln, insbesondere bevorzugt um eine wässrige Lösung aus Natriumhydroxid.

Eine weitere bevorzugte Ausführungsform ist dadurch gekennzeichnet, dass vor der Behandlung in der wässrigen Lösung eine mechanisch abtragende Oberflächenmodifikation in Form einer Strahlbehandlung erfolgt, insbesondere durch Sandstrahlung, vorzugsweise unter Verwendung von Aluminiumoxid-Partikeln mit einer durchschnittlichen Korngrösse von 0.05 - 0.25 mm oder 0.25 - 0.5 mm, insbesondere bevorzugt mit einem Druck zwischen 1 - 10 bar, vorzugsweise zwischen 1 - 6 bar, insbesondere bevorzugt zwischen 2- 5 bar. Alternativ oder zusätzlich ist es möglich, vor der Behandlung in der wässrigen Lösung und ggf. nach einer solchen mechanisch abtragenden Oberflächenmodifikation, eine chemische Oberflächenmodifikation durchzuführen, insbesondere durch Behandlung mit bevorzugt konzentrierter Schwefelsäure und/oder Salzsäure und/oder Flusssäure und/oder Salpetersäure oder Mischungen davon, bevorzugt bei einer Temperatur oberhalb von Raumtemperatur erfolgt.

Verfahren kann nach einer weiteren Ausführungsform dadurch gekennzeichnet sein, dass die wässrige Lösung vor der Verwendung als Tauchbad für das Implantat entgast wird, insbesondere derart, dass Carbonate aus der Lösung entfernt werden. Um diesen Carbonat-freien Zustand zu bewahren kann die Lösung bevorzugtermassen unter einer Schutzgasatmosphäre bis zur Verwendung gelagert werden.

Zur weiteren Verbesserung der Modifikation der Oberfläche kann zudem generell während der Behandlung wenigstens abschnittsweise in der Lösung Ultraschall angelegt werden. Unter Umständen reicht es, den Behälter mit der Lösung an die Wandung eines Ultraschall-Bades heranzuhalten, bevorzugt ist es, den Behälter mit der Lösung zum Tauchen des Implantates in ein Ultraschall-Bad einzutauchen. Generell zeigt es sich, dass es vorteilhaft ist, das Bad mechanischen Schwingungen auszusetzen während der Behandlung. Eine alternative Möglichkeit besteht darin, die üblicherweise bei Zahnärzten bereits in der Praxis vorhandenen piezoelektrischen Ultraschall-Handgeräte (beispielsweise für die Zahnreinigung) einzusetzen, sei es in dem sie in das Bad eingetaucht werden oder an den Behälter gehalten werden.

Alternativ oder zusätzlich ist es des weiteren möglich, das Implantat vor und/oder während und/oder nach der Behandlung in der Lösung mit UV-Licht zu bestrahlen.

Bei gewissen Materialien erweist es sich als nicht ganz einfach, die hydrophile Oberfläche auch nach längerer Lagerung nachhaltig gewährleisten zu können. Entsprechend betrifft die vorliegende Erfindung auch ein Verfahren, welches dadurch gekennzeichnet ist, dass ein Implantat, gegebenenfalls nach vorgängiger mechanisch und/oder chemisch abtragender Oberflächenmodifikation, und welches optional (aber keineswegs zwingend) bereits in einer wässrigen oder organischen Lösung von Alkali- Hydroxiden wie oben beschrieben behandelt worden ist, steril verpackt wird, und zusammen mit einem Behälter enthaltend eine wässrige oder organische Lösung von Alkali- -Hydroxiden, oder einer Mischung dieser Hydroxide, mit einer Gesamtkonzentration von Alkali- und/oder Erdalkali-Hydroxid im Bereich von 0.005M - 0.1M, wie sie oben beschrieben wurde, in einer Kombinationsverpackung verpackt wird. Dann kann erst kurz vor dem Einsetzen in den menschlichen Körper das Implantat nach Entnahme aus der Verpackung in dem Behälter in einem Verfahren wie oben beschrieben behandelt und dann eingesetzt werden.

Generell kann es von Vorteil sein, wenn die Lagerung des entweder bereits behandelten oder noch nicht behandelten Implantats im Dunkeln erfolgt. Entsprechend ist es bevorzugt, wenn der Behälter ein Behälter ist, in welchem das Implantat vor Licht geschützt ist.

Entsprechend betrifft die vorliegende Erfindung im Sinne eines kit of parts auch eine Kombinationspackung enthaltend wenigstens ein (steril) verpacktes Implantat, insbesondere bevorzugt ein Dentalimplantat, welches Implantat gegebenenfalls einer vorgängigen mechanisch und/oder chemisch abtragenden Oberflächenmodifikation unterzogen wurde, und/oder optional in einer wässrigen oder organischen Lösung von Alkali- -Hydroxiden wie weiter oben beschrieben behandelt wurde, sowie enthaltend wenigstens einen Behälter mit einer steril abgepackten wässrigen oder organischen Lösung von Alkali- -Hydroxiden, oder einer Mischung dieser Hydroxide, mit einer Gesamtkonzentration von Alkali-Hydroxid im Bereich von 0.005M - 0.1M, das heisst im wesentlichen mit einer Lösung, wie sie im Zusammenhang mit dem oben beschriebenen Verfahren verwendet wird. Bevorzugtermassen ist eine solche Kombinationspackung dadurch gekennzeichnet, dass der Behälter eine wässrige Lösung aus Natriumhydroxid in einer Konzentration von 0.008 - 0.1 Mol, bevorzugt von 0.01 - 0.1 Mol, insbesondere bevorzugt von 0.01 - 0.07 Mol enthält, wobei diese Lösung gegebenenfalls vor der Abfüllung zur Entfernung von CO₂ aus der Lösung mit einem Inertgas gespült wurde.

Des Weiteren betrifft die Erfindung ein metallisches Implantat mit einer hydrophilen Oberfläche zum wenigstens teilweisen Einsetzen in Hartgewebe wie in einen Knochen und/oder in Weichgewebe, dadurch gekennzeichnet, dass die hydrophile Metalloxid-Oberfläche wenigstens bereichsweise basisch, bevorzugt schwachbasisch modifiziert ist.

Des Weiteren betrifft die Erfindung ein metallisches Implantat mit einer hydrophilen Oberfläche zum wenigstens teilweisen Einsetzen in Hartgewebe wie in einen Knochen und/oder in Weichgewebe, herstellbar oder hergestellt nach einem Verfahren wie oben angegeben. Das Implantat besteht bevorzugt wenigstens an der Oberfläche oder bevorzugt vollständig aus Metalloxid und/oder Metall.

Das Implantat enthält bevorzugt Titan und/oder Titanoxid, welches gegebenenfalls zusätzlich mit Aluminium und Niob legiert ist, und/oder wobei diese Legierung statt Niob Vanadium enthält.

Bevorzugt ist das Implantat ein Dentalimplantat, dessen im implantierten Zustand dem Knochen und/oder Weichgewebe ausgesetzte Oberfläche basisch hydrophilisiert ist.

Die hydrophile Oberfläche ist bevorzugt wenigstens bereichsweise makrorau, insbesondere sandstrahlmodifiziert und mikrorau, insbesondere säuregeätzt.

Des Weiteren betrifft die Erfindung eine Verwendung eines solchen Implantates insbesondere hergestellt wie oben beschrieben als Dentalimplantat, insbesondere als Kronenstumpf, als Gewindestück, Schraube und/oder Stift.

Weitere bevorzugte Ausführungsformen der Erfindung sind in den abhängigen Ansprüchen beschrieben.

### KURZE ERLÄUTERUNG DER FIGUREN

Die Erfindung soll nachfolgend anhand von Ausführungsbeispielen im Zusammenhang mit den Abbildungen näher erläutert werden. Es zeigen:
- Fig. 1: die Abhängigkeit der Oberflächenhydrophilisierung (Kontaktwinkel) von der Konzentration der verwendeten basischen Lösung
- Fig. 2: die Abhängigkeit der Oberflächenhydrophilisierung (Kontaktwinkel) von der Konzentration der verwendeten basischen Lösung nach Lagerung an Luft ohne zusätzliche Stabilisierung
- Fig. 3: die Abhängigkeit der Oberflächenhydrophilisierung (Kontaktwinkel) von der Konzentration der verwendeten basischen Lösung nach Lagerung in basischer Umgebung;
- Fig. 4: die in vivo Resultate von mit zwei verschiedenen Material- und Oberflächenimplantattypen durchgeführten Versuchen;
- Fig. 5: XPS-Messungen an unbehandelter Probe (U) und an Vergleichsprobe (V) nach JP 2000-060958;
- Fig. 6: XPS-Messungen an NaOH-behandelten Proben gemäss der Erfindung;
- Fig. 7: XPS-Messungen an Sr(OH)₂-behandelten Proben gemäss der Erfindung;
- Fig. 8: XPS-Messungen im Detail im Bereich des Titan an Proben a) unbehandelt (U); b) gemäss JP 2000-060958 (V); c) behandelt mit NaOH nach der Erfindung; d) behandelt mit Sr(OH)₂ nach der Erfindung;
- Fig. 9: Raman-Spektren der Vergleichsproben nach JP 2000-060958;
- Fig. 10: Raman-Spektren an NaOH-behandelten Proben gemäss der Erfindung;
- Fig. 11: Raman-Spektren an Sr(OH)₂-behandelten Proben gemäss der Erfindung
- Fig. 12: vergleichende Raman-Spektren; und
- Fig. 13: Fotografien der Benetzung von Implantaten mit Blut, a) nicht erfindungsgemäss behandeltes Implantat, b) erfindungsgemäss behandeltes Implantat.

### WEGE ZUR AUSFÜHRUNG DER ERFINDUNG

Die vorliegende Erfindung beschreibt die Möglichkeit, die Oberfläche von Implantaten, die insbesondere aus metallischen Materialien gefertigt werden, chemisch zu modifizieren. Zweck der Oberflächenmodifizierung sind eine bessere Verankerung der Implantate im Hartgewebe, eine verbesserter Verbund zwischen Hartgewebe und Implantatoberfläche, ein besserer Verbund zwischen Weichgewebe und Implantatoberfläche und eine verbesserte Interaktion der Implantatoberfläche an der Grenzfläche Implantatoberfläche Hartgewebe und/oder Weichgewebe.

Vorzugsweise bezieht sich die Erfindung auf Implantate, welche im Hart- und/oder Weichgewebe verankert werden und dem temporären oder permanenten Ersatz oder Support von unfall-, abnutzungs-, mangelerscheinungs- oder krankheitsgeschädigten oder anderswie degenerierten Teilen des Bewegungsapparates unter Einschluss des Kauapparates, insbesondere des Dentalbereiches mit den zugehörigen auch ästhetischen Aspekten, dienen. So sind beispielsweise Hüft- und Kniegelenksprothesen, Wirbelsäulenimplantate und Dentalimplantate seit vielen Jahren im klinischen Einsatz. Die Aufgabe der verbesserten Osteointegrationseigenschaften respektive Osseointegrationseigenschaften wird erfindungsgemäss durch eine entsprechende Oberflächenbehandlung der (Metalloxid-)Oberfläche des Implantates gelöst, wobei die Behandlung sowohl über die gesamte Implantatoberfläche als auch von partiellen Bereichen der Implantatoberfläche erfolgen kann. Durch eine derartige Oberflächenbehandlung wird gewährleistet, dass die Metalle, wie vorzugsweise Titan und seine Legierungen, besser in das Hart- und /oder Weichgewebe integriert werden können.

Die strukturelle und funktionelle Verankerung, z. B. eines Zahnimplantats, im Knochen wird in der Regel durch Anbringen einer Makrorauhigkeit, und/oder einer, gegebenenfalls zusätzlichen, Mikrorauhigkeit, erreicht. Die Makrorauhigkeit kann beispielsweise mit einem mechanischen Strahlprozess, die Mikrorauhigkeit nachfolgend beispielsweise entweder in einem additiven Prozess mittels Plasmatechnik, oder in einem subtraktiven Prozess durch chemische Ätzung auf der Oberfläche bewirkt werden. Wie fest das Implantat im Knochen verankert ist, kann mit mechanischen Messungen festgestellt werden. Zahlreiche Untersuchungen haben gezeigt, dass genügende Verankerung eines Implantats im Knochen in hohem Mass von der Oberflächenbeschaffenheit des Implantats, insbesondere von der Rauhigkeit und der chemischen Umgebung an dessen Oberfläche, abhängt.

Die vorliegende Erfindung beschreibt eine spezifische und neuartig erzeugte (chemische Umgebung für eine) hydrophile Oberfläche zur besseren Osteointegration von Implantaten, welche aus Metallen, vorzugsweise aus Titan und seine Legierungen hergestellt sind. Diese erfindungsgemäss biologisch wirksame Oberfläche kann durch Verwendung einer basischen Lösung ggf. in Kombination beispielsweise mit zusätzlicher mechanischer Bearbeitung und Strukturierung, Kugelstrahlen, Sandstrahlen und/oder anschliessender oder vorgängiger chemischer Behandlung, beispielsweise Ätzung mit Säure o.ä. oder durch eine Kombination solcher Verfahren hergestellt werden.

Die erfindungsgemässe Oberfläche kann beispielsweise hergestellt werden, indem man die Oberfläche mit der gewünschten Rauhigkeit bzw. Textur versieht. Insbesondere kann man das Implantat herstellen, indem man die Implantatoberfläche kugelstrahlt, sandstrahlt und/oder unter Verwendung von 'Plasmatechnik strukturiert, und anschliessend die Oberfläche mit einem chemischen Prozess mit einer basischen Lösung behandelt, bis eine entsprechende hydrophile Oberfläche entstanden ist.

Wie erwähnt behandelt man das Implantat mit einer Base respektive einer wässrigen oder organischen basischen Lösung. Basen sind nach Brönsted definitionsgemäss Verbindungen, die Protonen aufnehmen. Nach Lewis sind Basen Moleküle oder Ionen mit einem einsamen Elektronennpaar oder einer elektronenreichen Mehrfachbindung Die Stärke von Basen kann beispielsweise über den pKb - Wert definiert werden. Basen oder basische Lösungen haben aber im vorliegenden Zusammenhang noch nie Verwendung gefunden zur stabilen Hydrophilisierung einer Implantatoberfläche im überraschenderweise ermittelten Konzentrationsbereich.

Es zeigt sich überraschenderweise, dass insbesondere bei Implantaten auf Basis von Metall basische Lösungen zu einer für die Integration in Knochen respektive Weichgewebe vorteilhaften hervorragenden Hydrophilisierung der Oberfläche führten.

Vorzugsweise wird die Oberfläche in der vorliegenden Anwendung mit einer Lösung von Natriumhydroxid hydrophilisiert. Es ist aber neben der Verwendung einer Lösung eines Hydroxides auch möglich, Lösungen auf Basis verschiedener Hydroxide zu verwenden.

Als besonders geeignet erweisen sich beispielsweise wässrige basische Lösungen bevorzugt aus Kaliumhydroxid oder Natriumhydroxid, wobei die Konzentration in einem Bereich von 0.0001 Mol bis 0.9 Mol, vorzugsweise im Bereich von 0.001 bis 0.1 vorgelegt wird. Als ganz besonders geeignet erweist es sich, wenn die Konzentration im Bereich 0.01 bis 0.07 M gewählt wird. Bei solchen schwachbasischen Lösungen insbesondere aus den genannten Komponenten wird bevorzugtermassen bei einer Temperatur im Bereich von - 10°C - 100 °C, insbesondere bei einer Temperatur im Bereich von 10°C-30 °C gearbeitet.

Generell kann gesagt werden, dass typischerweise eine basische Lösung bei einer Konzentration im Bereich von 0.001 - 0.09 M verwendet werden kann, vorzugsweise im Bereich von 0.01 - 0.09 M insbesondere bei einer Temperatur im Bereich von 0.01 - 0.07 M.

Gemäss einer weiteren bevorzugten Ausführungsform des erfindungsgemässen Verfahrens wird die Oberfläche wenigstens bereichsweise über eine Dauer von 2 Sekunden bis 1 Stunde, vorzugsweise von 5 Sekunden bis 10 Minuten, insbesondere von 5 Sekunden bis 1 Minute einer basischen Lösung, z. B. in Form eines Bades, ausgesetzt. Bevorzugtermassen wird eine Behandlungsdauer von weniger als einer Stunde, weiter bevorzugt von wenigstens fünf Sekunden, verwendet, um tatsächlich eine genügende Hydrophilisierung des Implantats durch die basischen Lösung gewährleisten zu können.

Mit derartig vorbehandelten Implantaten lässt sich ein sicherer Verbund zu Hart- und Weichgewebe herstellen.

### Experimentelle Herstellung von Implantaten:

### Beispiel 1

Eine gängige Form eines Zahnimplantats in Form einer Schraube von 3.5 mm Durchmesser und 10 mm Länge wurde aus Titan cp Grad 4 hergestellt. Die in den Knochen einzusetzende Oberfläche wurde nun mit einer Makrorauhigkeit versehen, indem diese mit einem Korn aus Al₂O₃ bei circa 4 bar sandgestrahlt wurde. Anschliessend wurde die aufgeraute Oberfläche mit einem Salzsäure-Schwefelsäure Gemisch heiss geätzt, um eine Mikrostrukturierung zu erhalten. Nach dem Ätzen wurde das Implantat mit reinem/deionisiertem Wasser behandelt und danach in deionisiertem Wasser gewaschen und gespült. Danach wurde das Implantat in eine wässrige 0.05 M NaOH-Lösung für 10 Sekunden getaucht. Nach dem Trocknen der Oberfläche an Luft konnte mittels vollständiger Benetzung mit einem Wassertropfen qualitativ festgestellt werden, dass sich die Oberfläche hydrophil verhält.

### Beispiel 2

Eine gängige Form eines Zahnimplantats in Form einer Schraube von 3.5 mm Durchmesser und 10 mm Länge wurde aus Titan cp Grad 4 hergestellt. Die in den Knochen einzusetzende Oberfläche wurde nun mit einer Makrorauhigkeit versehen, indem diese mit einem Korn aus Al₂O₃ bei circa 4 bar sandgestrahlt wurde. Anschliessend wurde die aufgerauhte Oberfläche mit einem Salzsäure-Schwefelsäure Gemisch heiss geätzt, um eine Mikrostrukturierung zu erhalten. Nach dem Ätzen wurde das Implantat mit reinem/deionisiertem Wasser behandelt und danach in deionisiertem Wasser gewaschen und gespült. Danach wurde das Implantat in eine wässrige 0.05 M NaOH-Lösung für 10 Sekunden getaucht. Nach dem Trocknen der Oberfläche an Luft wurde das Implantat 4 Wochen an Luft bei Raumtemperatur gelagert. Danach konnte mittels vollständiger Benetzung mit einem Wassertropfen qualitativ festgestellt werden, dass sich die Oberfläche hydrophil verhält.

### Beispiel 3

Eine gängige Form eines Zahnimplantats in Form einer Schraube von 3.5 mm Durchmesser und 10 mm Länge wurde aus Titan cp Grad 4 hergestellt. Die in den Knochen einzusetzende Oberfläche wurde nun mit einer Makrorauhigkeit versehen, indem diese mit einem Korn aus Al₂O₃ bei circa 4 bar sandgestrahlt wurde. Anschliessend wurde die aufgerauhte Oberfläche mit einem Salzsäure-Schwefelsäure Gemisch heiss geätzt, um eine Mikrostrukturierung zu erhalten. Nach dem Ätzen wurde das Implantat mit reinem/deionisiertem Wasser behandelt und danach in deionisiertem Wasser gewaschen und gespült. Danach wurde das Implantat in 0.05 M NaOH für 10 Sekunden getaucht. Danach wurde das Implantat 4 Wochen in eine wässrigen 0.01 M NaOH-Lösung bei Raumtemperatur gelagert. Die NaOH-Lösung wurde vorher mit N₂ gespült, um CO₂ aus der Lösung zu entfernen und damit während der Lagerung eine Karbonatbildung zu vermeiden. Danach konnte mittels vollständiger Benetzung mit einem Wassertropfen qualitativ festgestellt werden, dass sich die Oberfläche hydrophil verhält.

### Beispiel 4

Titan in Form von Plättchen mit 15 mm Durchmesser wurden aus Titan cp Grad 4 hergestellt. Die Oberfläche der Probekörper wurde nun mit einer Makrorauhigkeit versehen, indem diese mit einem Korn aus Al₂O₃ bei circa 4 bar sandgestrahlt wurde. Anschliessend wurde die aufgerauhte Oberfläche mit einem Salzsäure-Schwefelsäure Gemisch heiss geätzt, um eine Mikrostrukturierung zu erhalten. Nach dem Ätzen wurden die Probekörper mit reinem/deionisiertem Wasser behandelt und danach in deionisiertem Wasser gewaschen und gespült. Danach wurden die Probekörper in verschiedene wässrige Konzentrationen NaOH für ca. 10 Sekunden getaucht. Nach dem Trocknen der Oberfläche an Luft während 40 Minuten konnte mittels Kontaktwinkelmessungen quantitativ festgestellt werden, bei welcher Konzentration der Übergang vom hydrophoben zum hydrophilen Verhalten erfolgt.

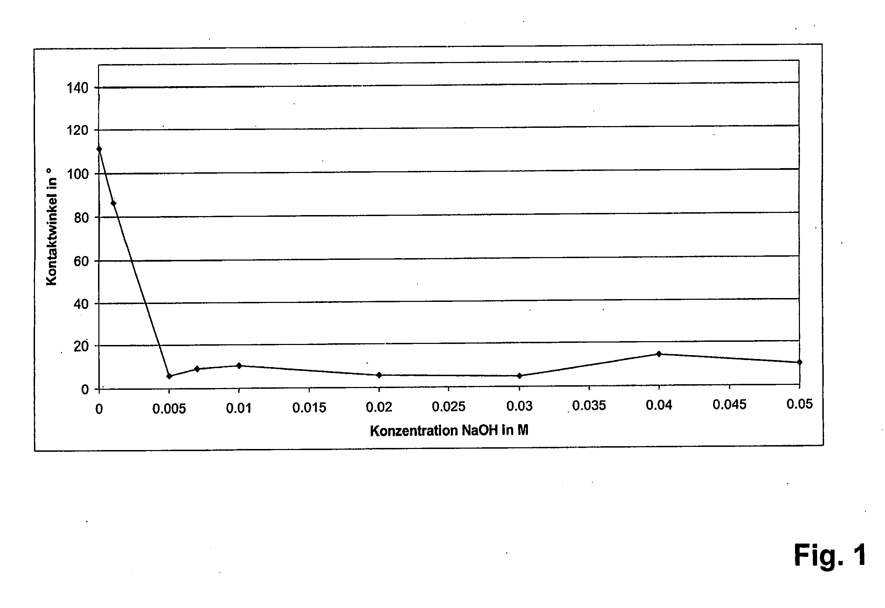

Figur 1 zeigt die Abhängigkeit der Oberflächenhydrophilisierung (Kontakwinkel) von der Konzentration der verwendeten basischen wässrigen Lösung. Es zeigt sich, dass beginnend bei völlig unerwartet tiefen Konzentrationen von ca. 0.005 M ein wesentlicher Effekt auftritt, und dass diese unerwartet tiefen Konzentrationen eine Behandlung ohne Nachbehandlung erlauben. Nachbehandlungen (Spülen, Hitze etc.) sind üblicherweise bei Konzentrationen von 1 M und mehr unabdingbar.

### Beispiel 5

Titan in Form von Plättchen mit 15 mm Durchmesser wurden aus Titan cp Grad 4 hergestellt. Die Oberfläche der Probekörper wurde nun mit einer Makrorauhigkeit versehen, indem diese mit einem Korn aus Al₂O₃ bei circa 4 bar sandgestrahlt wurde. Anschliessend wurde die aufgerauhte Oberfläche mit einem Salzsäure-Schwefelsäure Gemisch heiss geätzt, um eine Mikrostrukturierung zu erhalten. Nach dem Ätzen wurden die Probekörper mit reinem/deionisiertem Wasser behandelt und danach in deionisiertem Wasser gewaschen und gespült. Danach wurden die Probekörper in verschiedene wässrigen Konzentrationen NaOH für ca. 10 Sekunden getaucht. Nach dem Trocknen der Oberfläche an Luft wurde das Implantat 4 Wochen an Luft bei 30% Luftfeuchtigkeit bei Raumtemperatur im Dunkeln gelagert. Danach konnte mittels Kontaktwinkelmessungen quantitativ festgestellt werden, bei welcher Konzentration in Wasser der Übergang vom hydrophoben zum hydrophilen Verhalten erfolgt.

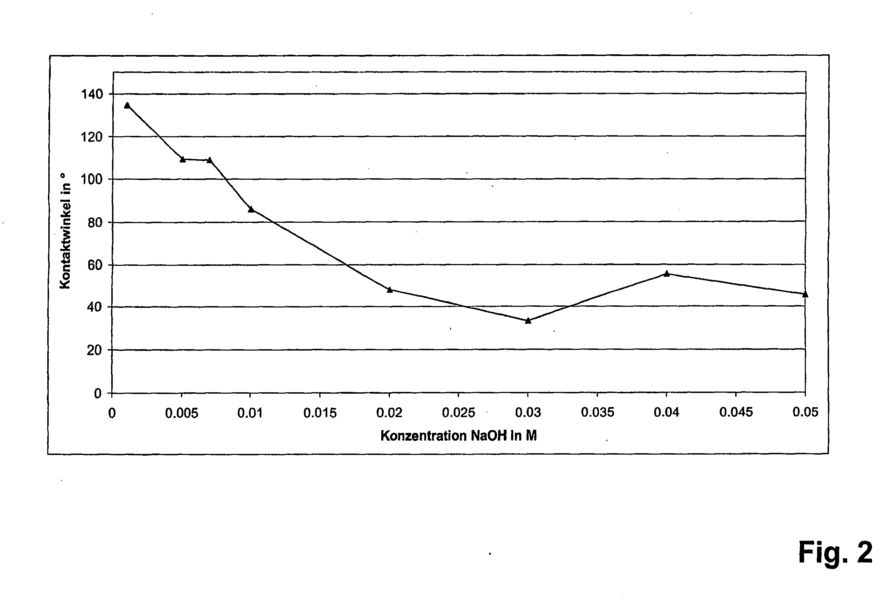

Figur 2 zeigt die Abhängigkeit der Oberflächenhydrophilisierung (Kontakwinkel) von der Konzentration der verwendeten basischen Lösung nach einer derartigen Lagerung an Luft.

### Beispiel 6

Titan in Form von Plättchen mit 15 mm Durchmesser wurden aus Titan cp Grad 4 hergestellt. Die Oberfläche der Probekörper wurde nun mit einer Makrorauhigkeit versehen, indem diese mit einem Korn aus Al₂O₃ bei circa 4 bar sandgestrahlt wurde. Anschliessend wurde die aufgerauhte Oberfläche mit einem Salzsäure-Schwefelsäure Gemisch heiss geätzt, um eine Mikrostrukturierung zu erhalten. Nach dem Ätzen wurden die Probekörper mit reinem/deionisiertem Wasser behandelt und danach in deionisiertem Wasser gewaschen und gespült. Danach wurden die Probekörper in verschiedene wässrigen Konzentrationen NaOH für ca. 10 Sekunden getaucht. Danach wurde das Implantat 4 Wochen in 0.01 M NaOH Raumtemperatur gelagert. Die wässrige NaOH-Lösung wurde vorher mit N₂ gespült, um CO₂ aus der Lösung zu entfernen und um während der Lagerung eine Karbonatbildung zu vermeiden. Die Proben wurden anschliessend aus dem Bad entnommen, und 40 Minuten getrocknet. Danach konnte mittels Kontaktwinkelmessungen quantitativ festgestellt werden, bei welcher Konzentration der Übergang vom hydrophoben zum hydrophilen Verhalten erfolgt.

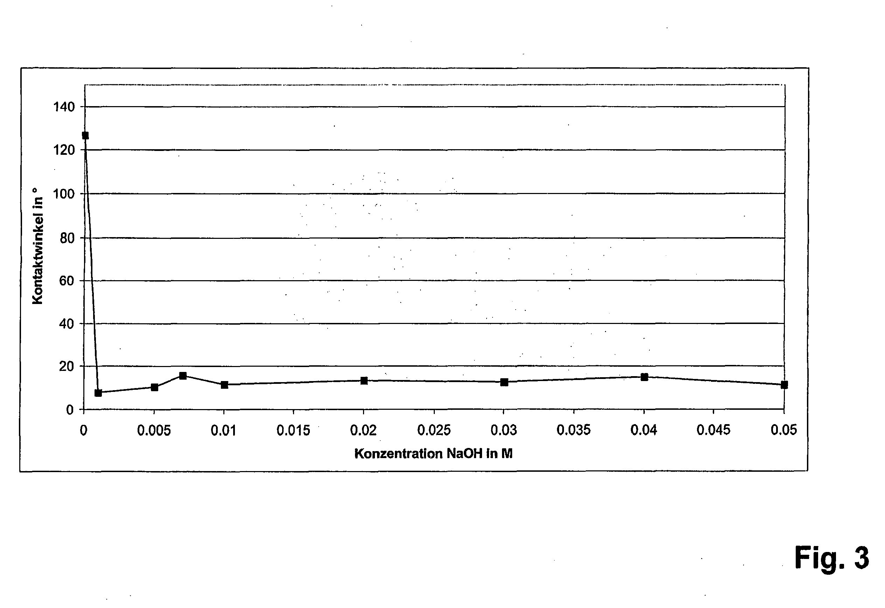

Figur 3 zeigt die Abhängigkeit der Oberflächenhydrophilisierung (Kontakwinkel) von der Konzentration der verwendeten basischen Lösung nach Lagerung in basischer Umgebung.

### Beispiel 7 (nicht nach der Erfindung)

Eine gängige Form eines Zahnimplantats in Form einer Schraube von 3.5 mm Durchmesser und 10 mm Länge wurde aus Zirkonoxid hergestellt. Die Rohform wurde aus einem zylindrischen keramischen Rohling in an sich bekannter Weise der mechanischen Keramikbearbeitung, hautsächlich durch Schleifen, erhalten. Die in den Knochen einzusetzende Oberfläche wurde nun mit einer Makrorauhigkeit versehen, indem diese mit einem Korn aus Al₂O₃ der mittleren Korngrösse 0.1-0.15 mm bei circa 3 bar sandgestrahlt wurde. Anschliessend wurde die aufgerauhte Oberfläche (Makrorauhigkeit) mit einem Kaliumhydroxid - Natriumhydroxid Gemisch in einer Salzschmelze mit einem Verhältnis von KOH : NaOH von 1 : 1 bei einer Temperatur von über 190° C während etwa 30 Stunden behandelt. Nach dem Ätzen wurde das Implantat im Ultraschall mit reinem/deionisiertem Wasser behandelt und danach in deionisiertem Wasser gewaschen und gespült.

Danach wurde das Implantat in eine wässrige 0.05 M NaOH-Lösung für 10 Sekunden getaucht. Danach wurde das Implantat 4 Wochen in 0.01 M NaOH bei Raumtemperatur gelagert. Die NaOH-Lösung wurde vorher mit N₂ gespült, um CO₂ aus der Lösung zu entfernen und damit während der Lagerung eine Karbonatbildung zu vermeiden. Danach konnte mittels vollständiger Benetzung mit einem Wassertropfen qualitativ festgestellt werden, dass sich die Oberfläche hydrophil verhält.

### Beispiel 8 (nicht nach der Erfindung)

Eine gängige Form eines Zahnimplantats in Form einer Schraube von 3.5 mm Durchmesser und 10 mm Länge wurde aus Zirkonoxid hergestellt. Die Rohform wurde aus einem zylindrischen keramischen Rohling in an sich bekannter Weise der mechanischen Keramikbearbeitung, hautsächlich durch Schleifen, erhalten. Die in den Knochen einzusetzende Oberfläche wurde nun mit einer Makrorauhigkeit versehen, indem diese mit einem Korn aus Al₂O₃ der mittleren Korngrösse 0.1-0.15 mm bei circa 3 bar sandgestrahlt wurde. Anschliessend wurde die aufgerauhte Oberfläche (Makrorauhigkeit) mit einem Kaliumhydroxid - Natriumhydroxid Gemisch in einer Salzschmelze mit einem Verhältnis von KOH : NaOH von 1 : 1 bei einer Temperatur von über 190° C während etwa 30 Stunden behandelt. Nach dem Ätzen wurde das Implantat im Ultraschall mit reinem/deionisiertem Wasser behandelt und danach in deionisiertem Wasser gewaschen und gespült. Nach dem Ätzen wurde das Implantat mit reinem/deionisiertem Wasser behandelt und danach in deionisiertem Wasser gewaschen und gespült.

Danach wurde das Implantat in eine wässrige 0.05 M NaOH-Lösung für 10 Sekunden getaucht. Nach dem Trocknen der Oberfläche an Luft wurde das Implantat 4 Wochen an Luft bei Raumtemperatur gelagert. Danach konnte mittels vollständiger Benetzung mit einem Wassertropfen qualitativ festgestellt werden, dass sich die Oberfläche hydrophil verhält.

### Beispiel 9

Titan Zahnimplantate wurden aus Titan cp Grad 4 hergestellt. Die Oberfläche der Implantate wurde nun mit einer Makrorauhigkeit versehen, indem diese mit einem Korn aus Al₂O₃ bei circa 4 bar sandgestrahlt wurde. Anschliessend wurde die aufgerauhte Oberfläche mit einem Salzsäure-Schwefelsäure Gemisch heiss geätzt, um eine Mikrostrukturierung zu erhalten. Nach dem Ätzen wurden die Implantate mit reinem/deionisiertem Wasser behandelt und danach in deionisiertem Wasser gewaschen und gespült. Anschliessend wurden die Implantate zunächst nicht weiter behandelt und individuell verpackt. Sie wurden anschliessend jeweils gemeinsam in einer Kombinationspackung verpackt mit einem separaten, steril verschlossenen Behälter enthaltend eine wässrige NaOH-Lösung mit unterschiedlichen Konzentrationen im Bereich von 0.005-0.5M, insbesondere 0.005-0.07M. Die Grösse und die Befüllung des Behälters war dabei derart gewählt, dass das Implantat nach dem Auspacken in den Behälter gelegt werden konnte, und anschliessend darin für einige Zeit gehalten werden konnte, ohne dass beim Eintauchen Lösung über den Rand trat und wobei das eingetauchte Implantat mit seinem zu behandelnden Bereich vollständig in die Lösung zu liegen kam. Diese Kombinationspackungen (kit of parts) wurden anschliessend während einer Dauer von mehreren Wochen gelagert.

Kurz vor der Verwendung (beispielsweise im Operationssaal) werden das Implantat ausgepackt und der Behälter geöffnet (Deckel abgezogen), und anschliessend das Implantat in den Behälter eingelegt, wobei es mit seinem zu behandelnden Bereich vollständig für ca. 10-30 Sekunden in die Lösung eintaucht. Nach dem (optionalen) Trocknen der Oberfläche an Luft während einigen Minuten konnte mittels vollständiger Benetzung mit einem Wassertropfen qualitativ festgestellt werden, dass sich die Oberfläche hydrophil verhält, und das noch feuchte oder getrocknete Implantat ist bereit für seine Einsetzung in den menschlichen Körper.

Es ist übrigens auch denkbar, dass die NaOH-Lösung in einer Ampulle in der Kombinationsverpackung vorliegt, und die Lösung zunächst in einen beim Endverbraucher zur Verfügung gestellten Behälter gekippt wird.

### Beispiel 10

Titan Zahnimplantate wurden aus Titan cp Grad 4 hergestellt. Die Oberfläche der Implantate wurde nun mit einer Makrorauhigkeit versehen, indem diese mit einem Korn aus Al₂O₃ bei circa 4 bar sandgestrahlt wurde. Anschliessend wurde die aufgerauhte Oberfläche mit einem Salzsäure-Schwefelsäure Gemisch heiss geätzt, um eine Mikrostrukturierung zu erhalten. Nach dem Ätzen wurden die Implantate mit reinem/deionisiertem Wasser behandelt und danach in deionisiertem Wasser gewaschen und gespült. Anschliessend wurden die Implantate zunächst nicht weiter behandelt und individuell verpackt. Sie wurden anschliessend jeweils gemeinsam in einer Kombinationspackung verpackt mit einem separaten, steril verschlossenen Behälter enthaltend eine wässrige NaOH-Lösung mit unterschiedlichen Konzentrationen im Bereich von 0.005-0.5M, insbesondere 0.005-0.07M. Die Grösse und die Befüllung des Behälters war dabei derart gewählt, dass das Implantat nach dem Auspacken in den Behälter gelegt oder gestellt oder an einem Instrument gehalten werden konnte, und anschliessend darin für einige Zeit gehalten werden konnte, ohne dass beim Eintauchen Lösung über den Rand trat und wobei das eingetauchte Implantat teilweise oder vollständig in die Lösung zu liegen kam. Diese Kombinationspackungen (kit of parts) wurden anschliessend während einer Dauer von mehreren Wochen gelagert. Kurz vor der Verwendung (beispielsweise im Operationssaal) werden das Implantat ausgepackt und der Behälter geöffnet (Deckel abgezogen), und anschliessend das Implantat in den Behälter eingelegt, wobei es teilweise oder vollständig für ca. 10-30 Sekunden in die Lösung eintaucht. Der NaOH enthaltende Behälter wird während der Behandlung einer Ultraschallanregung in einem Ultraschallbad und (gegebenenfalls generell mechanischen Schwingungen) ausgesetzt. Nach dem (optionalen) Trocknen der Oberfläche an Luft während einigen Minuten konnte mittels vollständiger Benetzung mit einem Wassertropfen qualitativ festgestellt werden, dass sich die Oberfläche hydrophil verhält, und das noch feuchte oder getrocknete Implantat bereit ist für seine Einsetzung in den menschlichen Körper.

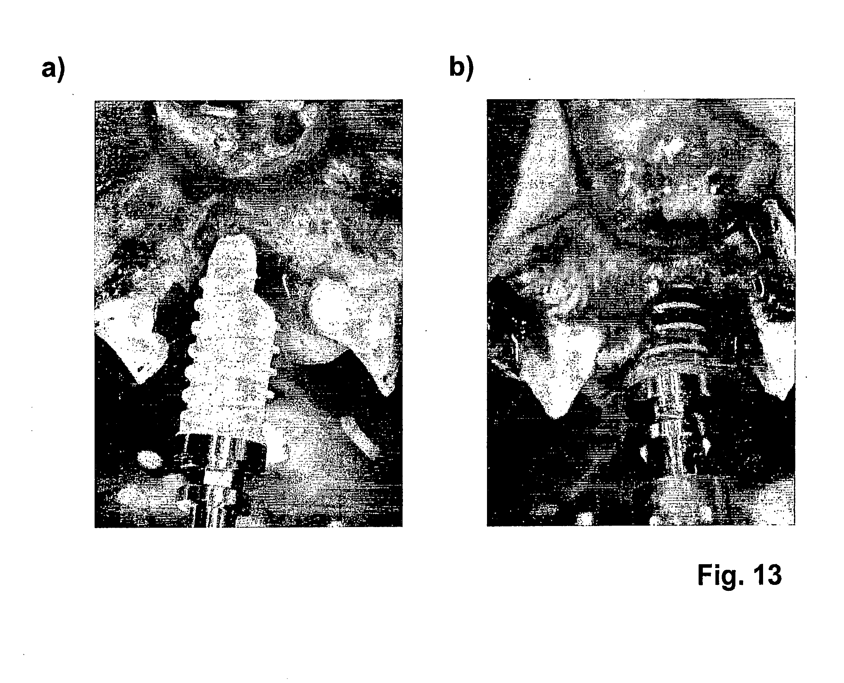

Bei diesen Proben wurde anhand der Benetzung mit Blut, die im Vergleich mit einem an der Oberfläche nicht behandelten Implantat fotografisch für jeweils nicht getrocknete Implantate festgehalten wurde (vergleiche Figur 13) die eindrückliche Veränderung der Oberfläche verifiziert.

### Beispiel 11

Titan Zahnimplantate wurden aus Titan cp Grad 4 gemäss Beispiel 10 hergestellt und nachbehandelt. Der NaOH enthaltende Behälter wird aber nun während der Behandlung nicht einer Ultraschallanregung in einem Ultraschallbad ausgesetzt sondern UV Bestrahlung ausgesetzt. Nach dem (optionalen) Trocknen der Oberfläche an Luft während einigen Minuten konnte mittels vollständiger Benetzung mit einem Wassertropfen qualitativ festgestellt werden, dass sich die Oberfläche hydrophil verhält, und das noch feuchte oder getrocknete Implantat bereit ist für seine Einsetzung in den menschlichen Körper.

### Beispiel 12

Eine gängige Form eines Zahnimplantats in Form einer Schraube von 3.5 mm Durchmesser und 10 mm Länge wurde aus Titan cp Grad 4 hergestellt und die in den Knochen einzusetzende Oberfläche wurde entsprechend Beispiel 10 behandelt. Nach dem (optionalen) Trocknen der Oberfläche an Luft während einigen Minuten konnte mittels vollständiger Benetzung mit einem Wassertropfen qualitativ festgestellt werden, dass sich die Oberfläche hydrophil verhält, und das noch feuchte oder getrocknete Implantat bereit ist für seine Einsetzung in den menschlichen Körper.

### Beispiel 13

Aus Titan cp Grad 4 wurden Scheiben mit einem Durchmesser von 15 mm hergestellt. Die einem Implantat vergleichbare, in den Knochen einzusetzende Oberfläche, wurde nun mit einer Makrorauhigkeit versehen, indem diese mit einem Korn aus Al₂O₃ bei circa 4 bar sandgestrahlt wurde. Anschliessend wurde die aufgerauhte Oberfläche mit einem Salzsäure-Schwefelsäure Gemisch heiss geätzt, um eine Mikrostrukturierung zu erhalten. Nach dem Ätzen wurde das Implantat mit reinem/deionisiertem Wasser behandelt und danach in deionisiertem Wasser gewaschen und gespült.

Danach wurde ein Teil der Titanscheiben entsprechend der eingangs diskutierten JP 2000-060958 im Sinne eines Vergleichsexperiments (V) behandelt. Dazu wurden die Proben in eine Mischung aus 1.5 M NaOH, 1.5 M KOH im Verhältnis 1:1 für 24 h bei 50°C in ein verschlossenes Gefäss eingetaucht und danach bei 200°C für 3 Stunden kalziniert. Nachdem dem Abkühlen der Proben wurden diese entsprechend der JP 2000-060958 in 10 mM Ca(OH)₂ Lösung bei 80°C für 1 h eingetaucht, danach kurz in DI Wasser abgespült und getrocknet. An den so nach JP 2000-060958 behandelten Proben wurden XPS und ramanspektroskopische Untersuchungen vorgenommen.

Der andere Teil der, wie oben beschrieben, präparierten Titanscheiben wurde entsprechend der erfindungsgemässen Behandlung (vgl. z.B. Beispiel 10) unterzogen. Dazu wurden je sechs Titanscheiben mit NaOH der Konzentrationen 0.01; 0.05; 0.005 M für jeweils 10 Sekunden behandelt (eingetaucht). Weiterhin wurden je sechs Titanscheiben mit Sr(OH)₂ der Konzentrationen für jeweils 10 behandelt (eingetaucht, nicht nach der Erfindung). Das Gefäss mit der NaOH bzw. Sr(OH)₂ Lösung befand sich dazu jeweils in einem eingeschalteten Ultraschallbad. An den so erfindungsgemäss behandelten Proben wurden ebenfalls XPS und ramanspektroskopische Untersuchungen vorgenommen und mit den Ergebnissen der nach JP 2000-060958 behandelten Proben verglichen.

Die Untersuchungen zeigen die erwarteten deutlichen Unterschiede zwischen der erfindungsgemäss hydrophilisierenden und der chemisch und strukturell oberflächenverändemden Behandlung nach JP 2000-060958. Die Quantifizierung der XPS Messungen, jeweils jede Probe an zwei verschiedenen Stellen ausgemessen, zeigt eindeutige Unterschiede in der chemischen Zusammensetzung der Oberfläche, siehe nachfolgende Tabelle.

**Tabelle 1: Quantitative Auswertung der XPS Messungen**

| **Proben** JP 2000-060958 | | | | | | | |
|---|---|---|---|---|---|---|---|
| **Atomkonzentration [%]** | | | | | | | |
| | **C 1s** | **Ca 2p** | **Mg 2p** | **Na KLL** | **O 1s** | **Ti 2p** | |
| V Probe 1 | 26.3 | 6.5 | 2.9 | 0.4 | 52.3 | 11.7 | |
| V Probe 1 | 27.9 | 6.3 | 2.0 | 0.4 | 51.1 | 12.2 | |
| V Probe 2 | 27.4 | 6.6 | 2.3 | 0.4 | 52.1 | 11.2 | |
| V Probe 2 | 26.6 | 6.2 | 3.7 | 0.3 | 52.4 | 10.8 | |

| **Proben NaOH** | | | | | | | |
|---|---|---|---|---|---|---|---|
| **Atomic Concentration [%]** | | | | | | | |
| | **C 1s** | **N 1s** | **Na KLL** | **O 1s** | **Ti 2p** | **Zn 2p** | |
| NaOH 0.01M Probe 1 | 18.6 | 2.7 | 8.5 | 49.1 | 20.7 | 0.4 | |
| NaOH 0.01M Probe 1 | 18.3 | 1.8 | 8.7 | 50.4 | 20.3 | 0.5 | |
| NaOH 0.01M Probe 2 | 18.5 | 1.9 | 9.0 | 50.5 | 19.8 | 0.4 | |
| NaOH 0.01M Probe 2 | 19.3 | 2.5 | 8.4 | 48.6 | 20.8 | 0.3 | |
| NaOH 0.05M Probe 1 | 18.8 | 1.9 | 10.2 | 49.7 | 19.0 | 0.3 | |
| NaOH 0.05M Probe 1 | 18.6 | 2.1 | 10.9 | 49.9 | 18.1 | 0.4 | |
| NaOH 0.05M Probe 2 | 19.2 | 1.8 | 13.7 | 49.2 | 15.9 | 0.2 | |
| NaOH 0.05M Probe 2 | 19.6 | 1.8 | 13.4 | 49.1 | 15.9 | 0.2 | |
| NaOH 0.005M Probe 1 | 20.3 | 2.0 | 8.6 | 49.9 | 18.6 | 0.6 | |
| NaOH 0.005M Probe 1 | 20.4 | 1.6 | 6.7 | 49.9 | 20.9 | 0.5 | |
| NaOH 0.005M Probe 2 | 18.3 | 2.4 | 6.0 | 51.4 | 21.7 | 0.4 | |
| NaOH 0.005M Probe 2 | 19.3 | 2.4 | 6.0 | 50.0 | 22.1 | 0.2 | |

| **Proben Sr(OH)2** | | | | | | | |
|---|---|---|---|---|---|---|---|
| **Atomic Concentration [%]** | | | | | | | |
| | **C 1s** | **Ca 2p** | **N 1s** | **O 1s** | **Sr 3d** | **Ti 2p** | **Zn 2p** |
| Sr(OH)2 0.01M Probe 1 | 24.2 | 2.0 | 2.8 | 51.0 | 6.0 | 13.6 | 0.5 |
| Sr(OH)2 0.01M Probe 1 | 17.1 | 0.7 | 2.4 | 52.8 | 2.3 | 24.3 | 0.5 |
| Sr(OH)2 0.01M Probe 2 | 19.0 | 0.4 | 3.5 | 50.5 | 2.2 | 24.2 | 0.3 |
| Sr(OH)2 0.01M Probe 2 | 18.5 | 0.3 | 2.3 | 52.4 | 2.5 | 23.8 | 0.3 |
| Sr(OH)2 0.05M Probe 1 | 15.5 | 1.5 | 2.3 | 56.0 | 5.1 | 19.6 | 0.0 |
| Sr(OH)2 0.05M Probe 1 | 18.5 | 1.6 | 1.9 | 53.4 | 7.1 | 17.2 | 0.3 |
| Sr(OH)2 0.05M Probe 2 | 14.6 | 1.3 | 1.5 | 57.9 | 5.2 | 19.2 | 0.3 |
| Sr(OH)2 0.05M Probe 2 | 19.8 | 1.6 | 1.3 | 52.9 | 7.0 | 17.4 | 0.0 |
| Sr(OH)2 0.005M Probe 1 | 17.4 | 0.6 | 2.8 | 52.4 | 1.7 | 24.8 | 0.4 |
| Sr(OH)2 0.005M Probe 1 | 17.3 | 0.4 | 2.3 | 52.5 | 1.8 | 24.8 | 0.8 |
| Sr(OH)2 0.005M Probe 2 | 20.5 | 0.5 | 3.0 | 50.1 | 2.1 | 23.2 | 0.6 |
| Sr(OH)2 0.005M Probe 2 | 19.4 | 0.8 | 2.8 | 50.8 | 2.9 | 22.8 | 0.5 |

Die erfindungsgemäss behandelten Proben zeigen an der Oberfläche wesentlich mehr Titan als die nach JP 2000-060958 behandelten Proben und entsprechend der Behandlung einen wesentlichen Anteil an Na respektive Sr an der Oberfläche, welcher an der Oberfläche der Titanscheiben nach JP 2000-060958 nicht vorhanden ist.

Dagegen zeigen die nach JP 2000-060958 behandelten Proben einen wesentlich höheren Ca Anteil an der Oberfläche.

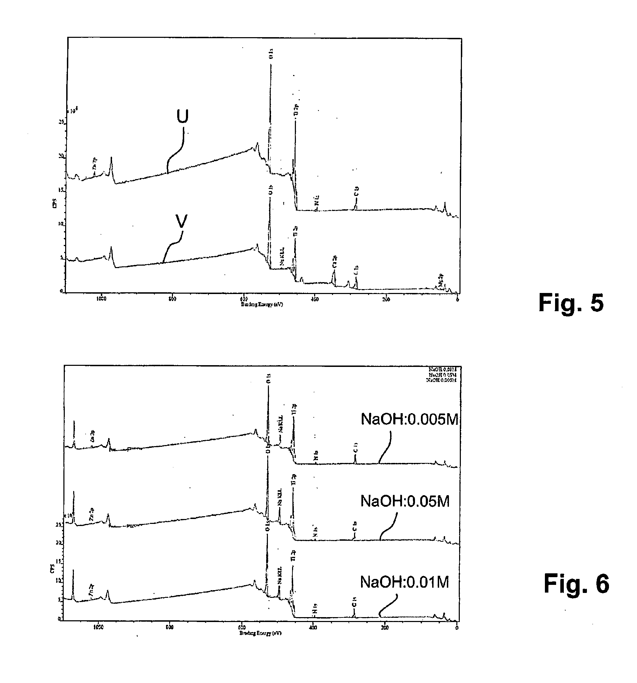

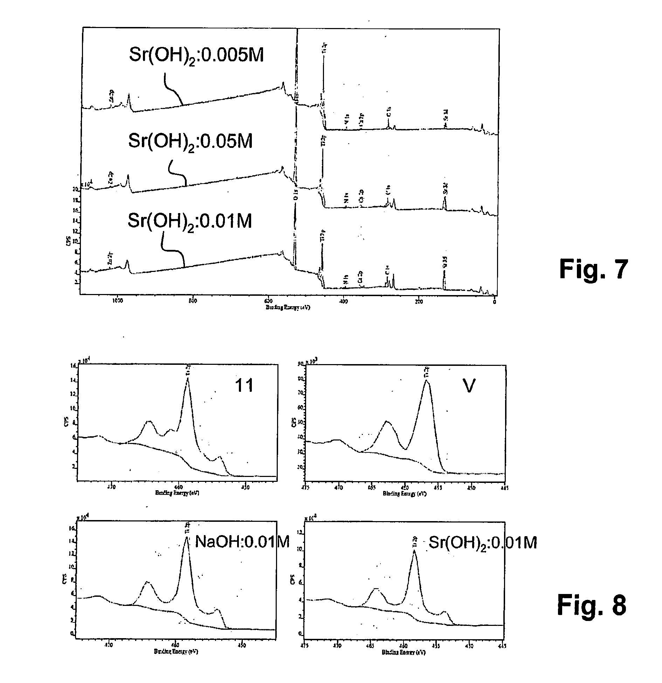

Die XPS-Spektren selbst sind in den Figuren 5 bis 8 dargestellt. Figur 5 zeigt das Spektrum der nach JP 2000-060958 behandelten Proben im Vergleich zu einer nicht hydrophilisierten (unbehandelten) Probe. Figur 6 zeigt die Spektren der erfindungsgemäss mit NaOH behandelten Proben und Figur 7 die der erfindungsgemäss mit Sr(OH)₂ behandelten Proben. Klar ersichtlich sind die schon in der Quantifizierung deutlichen Unterschiede bezüglich Ca und weiterhin die verschiedenen Bindungsenergien für Titan.

Daher wurden zusätzlich die Detailspektren für das Titan gemessen. In Figur 8 ist deutlich sichtbar, dass die Oberflächenbehandlung nach JP 2000-060958 zu einer Änderung in der chemischen Umgebung von Titan führt.

Entgegengesetzt dazu zeigen die erfindungsgemäss oberflächenbehandelten Proben keine Änderung in der chemischen Umgebung des Titans zu der in der Abbildung ebenfalls dargestellten nicht hydrophilisierten (unbehandelten) Oberfläche.

Untersuchungen mittels Ramanspektroskopie zeigen ebenfalls Unterschiede zwischen der erfindungsgemäss hydrophilisierenden und der chemisch und strukturell oberflächenverändernden Behandlung nach JP 2000-060958.

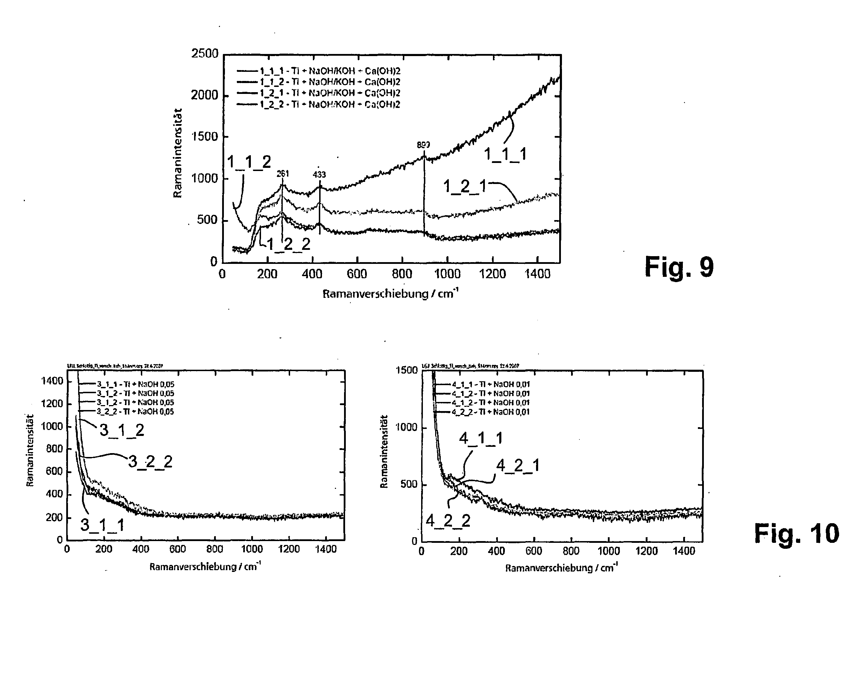

In den Spektren der Vergleichsproben 1-1 und 1-2, Figur 9, (JP 2000-060958 Behandlung) werden die breiten Peaks bei 261 cm-1, 433 cm-1 und 663 cm-1 sowie die Stufe bei 899 cm-1 beobachtet. Diese können nicht den kristallinen Phasen des Titanoxides (Anatas, Rutil oder Brookit) und auch nicht eindeutig Calciumcarbonat, Calciumtitanat oder sogar Apatit (entgegen der Aussage in der JP 2000-060958) zugeordnet werden. Es handelt sich wahrscheinlich um amorphes Titanoxid.

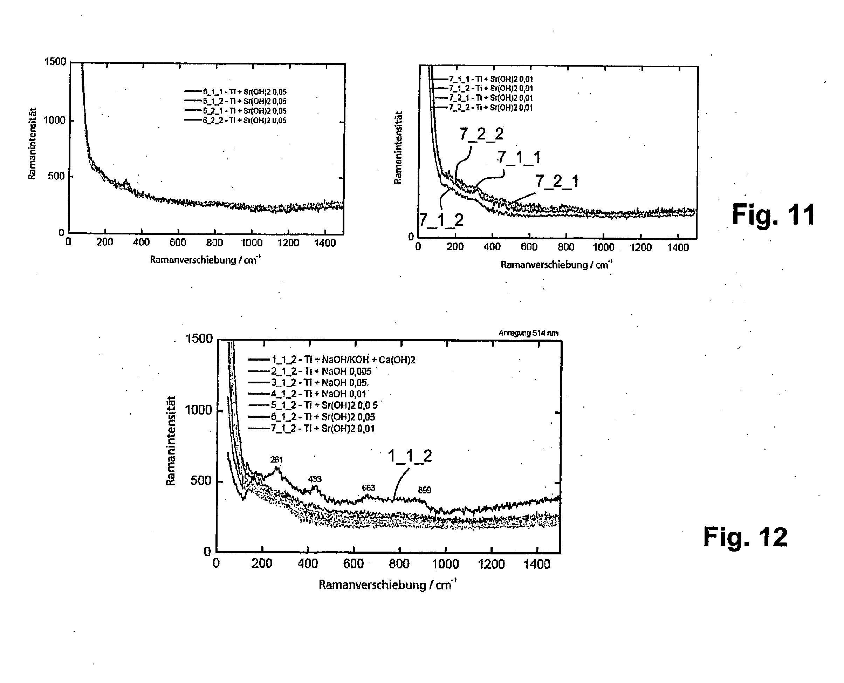

Im Gegensatz dazu werden in den Spektren der hydrophilisierten Proben gar keine Ramanpeaks detektiert, vgl. Figuren 10 und 11. Es können also keine Raman-aktive Verbindungen auf der Oberfläche der Proben nachgewiesen werden.

Die Ramanspektren der Proben behandelt nach JP 2000-060958 unterscheiden sich generell von den Ramanspektren aller hydrophilisierten Proben, die untereinander vergleichbar sind, vgl. Figur 12.

### In vivo-Tests:

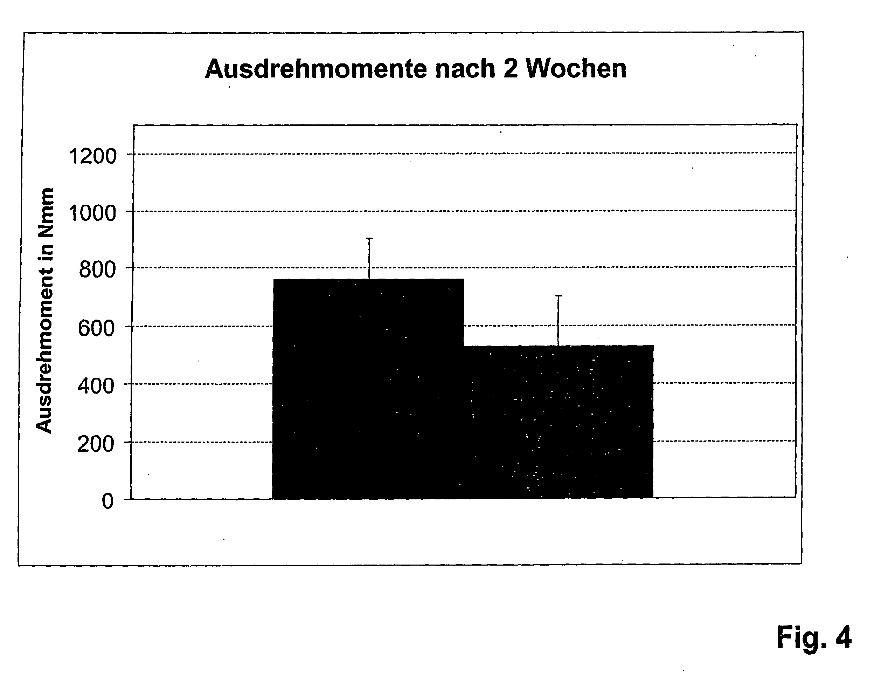

Es zeigte sich bei sämtlichen beispielhaft hergestellten Implantaten mit hydrophiler Oberfläche ( Beispiele 1 bis 13 ), dass die Osseointegration resp. die Osteointegration gut erfolgte. Weiterhin zeigt sich auch eine gute Integration an Weichgewebe (z. B. Zahnfleisch). Die Figur 4 zeigt entsprechende Resultate von mit zwei verschiedenen Material und Oberflächenimplantattypen durchgeführten Versuchen. Dabei wurde ein Titanimplantat mit einem Durchmesser von 4.2 mm und einer Länge von 8 mm mit einer erfindungsgemäss hergestellten Oberfläche im wesentlichen gemäss dem oben beschriebenen Beispiel 1 (Messung 1 in Figur 4, links) und ein entsprechend dimensioniertes Dentalimplantat mit einer plasmachemisch anodisch oxidierten Oberfläche (Messung 2 in Figur 4, rechts) eines Titanimplantates verglichen. Die plasmachemisch anodisch oxidierte Oberfläche entspricht der Oberfläche von kommerziell sehr verbreiteten und häufig verwendeten Implantaten. Die Implantate wurden in die Beckenschaufel von Schafen implantiert. Nach einer Einheilzeit von 2 Wochen wurde das Ausdrehmoment bestimmt, welches erforderlich war, um die eingewachsenen Implantate vom Knochen zu lösen. Wie Figur 4 zeigt, findet man ein besseres Einwachsen des neuen Implantates.

Histologische Messungen zeigen zudem eindeutig bessere Kontaktflächen (BIC, bone to implant contact) im Vergleich zu den nicht nach der Erfindung behandelten Implantaten.

## Patentansprüche

1. Verfahren zur Herstellung eines metallischen Implantats mit einer hydrophilen Oberfläche zum wenigstens teilweisen Einsetzen in Hartgewebe wie in einen Knochen und/oder in Weichgewebe, **dadurch gekennzeichnet, dass** das Verfahren wenigstens einen Schritt umfasst, in welchem wenigstens ein zum teilweisen Einsetzen in Hartgewebe wie in einen Knochen und/oder in Weichgewebe vorgesehener Bereich, gegebenenfalls nach vorgängiger mechanisch und/oder chemisch abtragender Oberflächenmodifikation, in einer wässrigen oder organischen Lösung von Alkali -Hydroxiden, oder einer Mischung dieser Hydroxide, mit einer Gesamtkonzentration von Alkali-Hydroxid im Bereich von 0.005M - 0.1M während einer Zeitspanne im Bereich von 2sec - 10 min behandelt wird, wobei in dem Schritt der Alkali-Hydroxid-Behandlung Metalloxide an der Oberfläche modifiziert werden, und wobei die Behandlung in einer wässrigen Lösung von Alkali-Hydroxiden, oder einer Mischung dieser Hydroxide, bei einer Temperatur im Bereich von 10 - 30° stattfindet.

2. Verfahren nach Anspruch 1, **dadurch gekennzeichnet, dass** es sich um eine wässrige oder organische Lösung von einem oder mehreren Alkali-Hydroxiden, handelt, wobei eine Gesamtkonzentration im Bereich von Bereich von 0.05M - 0.1M verwendet wird.

3. Verfahren nach Anspruch 1 oder 2, **dadurch gekennzeichnet, dass** es sich bei der basischen Lösung um eine wässrige Lösung ausschliesslich bestehend aus einem oder mehreren der genannten Hydroxide handelt.

4. Verfahren nach einem der vorhergehenden Ansprüche, **dadurch gekennzeichnet, dass** in einer wässrigen Lösung von Alkali-Hydroxiden, oder einer Mischung dieser Hydroxide, mit einer Gesamtkonzentration von Alkali-Hydroxid im Bereich von 0.05M - 0.1M kurzzeitig behandelt wird.

5. Verfahren nach einem der vorhergehenden Ansprüche, **dadurch gekennzeichnet, dass** die Behandlung während einer Zeitspanne im Bereich von 5sec - 120 sec, bevorzugt von 5 - 30 sec stattfindet.

6. Verfahren nach einem der vorhergehenden Ansprüche, **dadurch gekennzeichnet, dass** es sich bei der wässrigen Lösung um eine Lösung aus Natriumhydroxid handelt, bevorzugt um eine Lösung aus Natriumhydroxid in einer Konzentration von 0.008 - 0.4 M, bevorzugt von 0.01 - 0.1 M, insbesondere bevorzugt von 0.01 - 0.07 M, wobei bevorzugtermassen bei Raumtemperatur gearbeitet wird.

7. Verfahren nach einem der vorhergehenden Ansprüche, **dadurch gekennzeichnet, dass** die Implantatoberfläche nach der Behandlung mit der wässrigen Lösung ohne Nachbehandlung mit erhöhter Temperatur und/oder Nachbehandlung durch Spülen gelagert und/oder verpackt wird.

8. Verfahren nach einem der vorhergehenden Ansprüche, **dadurch gekennzeichnet, dass** die wässrige Lösung vor der Verwendung als Tauchbad für das Implantat entgast wird, und gegebenenfalls unter einer Schutzgasatmosphäre bis zur Verwendung gelagert wird.

9. Verfahren nach einem der vorhergehenden Ansprüche, **dadurch gekennzeichnet, dass** während der Behandlung wenigstens abschnittsweise in der Lösung Ultraschall angelegt wird, und/oder dass das Implantat vor und/oder während und/oder nach der Behandlung in der Lösung mit UV-Licht bestrahlt wird.

10. Verfahren nach einem der vorhergehenden Ansprüche, **dadurch gekennzeichnet, dass** die Implantatoberfläche nach der Behandlung mit der wässrigen Lösung in einer basischen Lösung gelagert und verpackt wird, wobei es sich bevorzugt bei der basischen Lösung um eine Lösung von bevorzugt ausschliesslich Alkali- Hydroxiden, oder eine Mischung dieser Lösungen handelt, insbesondere um eine Lösung aus Natriumhydroxid, bevorzugt in einer Konzentration von 0.0001 - 0.9 M.

11. Verfahren nach einem der vorhergehenden Ansprüche, **dadurch gekennzeichnet, dass** vor der Behandlung in der wässrigen Lösung eine mechanisch abtragende Oberflächenmodifikation in Form einer Strahlbehandlung erfolgt, insbesondere durch Sandstrahlung, vorzugsweise unter Verwendung von Aluminiumoxid-Partikeln mit einer durchschnittlichen Korngrösse von 0.05 - 0.25 mm oder 0.25 - 0.5 mm, insbesondere bevorzugt mit einem Druck zwischen 1 - 10 bar, vorzugsweise zwischen 1 - 6 bar, insbesondere bevorzugt zwischen 2- 5 bar.

12. Verfahren nach einem der vorhergehenden Ansprüche, **dadurch gekennzeichnet, dass** vor der Behandlung in der wässrigen Lösung und ggf. nach einer mechanisch abtragende Oberflächenmodifikation nach Anspruch 11, eine chemische Oberflächenmodifikation erfolgt, insbesondere durch Behandlung mit bevorzugt konzentrierter Schwefelsäure und/oder Salzsäure und/oder Flusssäure und/oder Salpetersäure oder Mischungen davon bevorzugt bei einer Temperatur oberhalb von Raumtemperatur erfolgt.

13. Verfahren nach einem der vorhergehenden Ansprüche, **dadurch gekennzeichnet, dass** das Implantat, gegebenenfalls nach vorgängiger mechanisch und/oder chemisch abtragender Oberflächenmodifikation, und welches optional bereits in einer wässrigen oder organischen Lösung von Alkali-Hydroxiden behandelt worden ist, steril verpackt wird, und zusammen mit einem Behälter enthaltend eine wässrige oder organische Lösung von Alkali-Hydroxiden, oder einer Mischung dieser Hydroxide, mit einer Gesamtkonzentration von Alkali-Hydroxid im Bereich von 0.005M - 0.1M, in einer Kombinationsverpackung verpackt wird, und erst kurz vor dem Einsetzen in den menschlichen Körper das Implantat nach Entnahme aus der Verpackung in dem Behälter nach einem der vorhergehenden Ansprüche behandelt wird.

14. Kombinationspackung
enthaltend wenigstens ein steril verpacktes Implantat, insbesondere bevorzugt ein Dentalimplantat, welches Implantat gegebenenfalls einer vorgängigen mechanisch und/oder chemisch abtragenden Oberflächenmodifikation unterzogen wurde, und/oder optional in einer wässrigen oder organischen Lösung von Alkali-Hydroxiden nach einem der Ansprüche 1-9 behandelt wurde, sowie
enthaltend wenigstens einen Behälter mit einer steril abgepackten, gegebenenfalls entgasten und optional unter Schutzgas gehaltenen, wässrigen oder organischen Lösung von Alkali-Hydroxiden, oder einer Mischung dieser Hydroxide, mit einer Gesamtkonzentration von Alkali-Hydroxid im Bereich von 0.005M-0.1M.

15. Kombinationspackung nach Anspruch 14, **dadurch gekennzeichnet, dass** der Behälter eine Lösung aus Natriumhydroxid in einer Konzentration von 0.008 - 0.1 Mol, bevorzugt von 0.01 - 0.1 Mol, insbesondere bevorzugt von 0.01 - 0.07 Mol enthält, wobei diese Lösung gegebenenfalls vor der Abfüllung zur Entfernung von CO₂ aus der Lösung mit einem Inertgas gespült wurde.

16. Metallisches Implantat mit einer hydrophilen Oberfläche zum wenigstens teilweisen Einsetzen in Hartgewebe wie in einen Knochen und/oder in Weichgewebe, **dadurch gekennzeichnet, dass** die hydrophile Metalloxid-Oberfläche wenigstens bereichsweise basisch, bevorzugt schwachbasisch modifiziert ist, herstellbar oder hergestellt nach einem Verfahren nach einem der Ansprüche 1 - 13, wobei dieses bevorzugt wenigstens an der Oberfläche oder bevorzugt vollständig aus Metalloxid und/oder Metall besteht und/oder dass dieses bevorzugt Titan und/oder Titanoxid enthält, welches gegebenenfalls zusätzlich mit Aluminium und Niob legiert ist, und/oder dass diese Legierung bevorzugt statt Niob Vanadium enthält, und/oder dass es sich um bevorzugt ein Dentalimplantat handelt, dessen im implantierten Zustand dem Knochen und/oder Weichgewebe ausgesetzte Oberfläche basisch hydrophilisiert ist, und/oder dass die hydrophile Oberfläche bevorzugt wenigstens bereichsweise makrorau, insbesondere sandstrahlmodifiziert und mikrorau, insbesondere säuregeätzt ist.

## Claims

1. Method for the production of a metallic implant with a hydrophilic surface for at least partial insertion into hard tissue such as into a bone and/or into soft tissue, wherein the method comprises at least one step, in which at least one area, which is provided for partial insertion into hard tissue as into a bone and/or into soft tissue, is subjected, possibly after an antecedent mechanically and/or chemically abrasive surface modification, to a short-time treatment in an aqueous or organic solution of alkali-hydroxides, or a mixture of these hydroxides, with a total concentration of alkali-hydroxide in the range of 0.005M-0.1M, wherein the treatment is carried out during a time span in the range of 2sec-10min, wherein in the step of the alkali-hydroxide treatment, metal oxides are modified at the surface, and wherein the treatment is carried out in an aqueous solution of alkali-hydroxides, or a mixture of these hydroxides, at a temperature in the range of 10-30°.

2. Method according to claim 1, wherein the solution is an aqueous or organic solution of one or more alkali-hydroxides, wherein a total concentration in the range of 0.05M-0.1M is used.

3. Method according to one of the preceding claims 1 or 2, wherein the alkaline solution is an aqueous solution consisting exclusively of one or more of the named hydroxides.

4. Method according to one of the preceding claims, wherein the short-term treatment is carried out in an aqueous solution of alkali-hydroxides, or a mixture of these hydroxides, with a total concentration of alkali- hydroxide in the range of 0.05M-0.1M.

5. Method according to one of the preceding claims, wherein the treatment is carried out during a time span in the range of 5sec-120sec, preferably of 5-30sec.

6. Method according to one of the preceding claims, wherein the aqueous solution is a solution of sodium hydroxide, preferably a solution of sodium hydroxide at a concentration of 0.008-0.4 M, preferably of 0.01-0.1 M, especially preferably of 0.01-0.07 M, wherein the treatment is carried out preferably at room temperature.

7. Method according to one of the preceding claims, wherein the implant surface is stored and/or packaged after the treatment with the aqueous solution without any post-treatment with an increased temperature and/or any post-treatment by rinsing.

8. Method according to one of the preceding claims, wherein the aqueous solution is degassed prior to its use as an immersion bath for the implant, and possibly is stored in an inert gas atmosphere until use.

9. Method according to one of the preceding claims, wherein during the treatment ultrasound is applied to the solution at least period-wise, and/or wherein the implant is irradiated with UV-light prior to and/or during and/or after the treatment in the solution.

10. Method according to one of the preceding claims, wherein the implant surface is stored and packaged in an alkaline solution after the treatment with the aqueous solution, wherein preferably the alkaline solution is a solution of preferably exclusively alkali-hydroxides, or a mixture of these solutions, wherein in particular the alkaline solution is a solution of sodium hydroxide, preferably at a concentration of 0.0001-0.9 M.

11. Method according to one of the preceding claims, wherein prior to the treatment in the aqueous solution, a mechanically abrasive surface modification is carried out in the form of a blasting-treatment, preferably by sand blasting, preferably by the use of aluminium oxide particles with an average particle size of 0.05-0.25 mm or 0.25-0.5 mm, especially preferably with a pressure between 1-10 bar, preferably between 1-6 bar, especially preferably between 2-5 bar.

12. Method according to one of the preceding claims, wherein prior to the treatment in the aqueous solution and possibly after a mechanically abrasive surface modification according to claim 11, a chemical surface modification is carried out, preferably by treatment with preferably concentrated sulphuric acid and/or hydrochloric acid and/or hydrofluoric acid and/or nitric acid or mixtures thereof, preferably at a temperature above room temperature.

13. Method according to one of the preceding claims, wherein the implant, possibly after a prior mechanically and/or chemically abrasive surface modification, and which optionally has already been treated in an aqueous or organic solution of alkali- and/or alkaline-earth-hydroxides, is packaged sterilely, and is packaged in a combination package together with a container which contains an aqueous or organic solution of alkali-hydroxides, or a mixture of these hydroxides, with a total concentration of alkali-hydroxide in the range of 0.005M-0.1M, and wherein the implant is treated in the container by a method according to one of the preceding claims after release from the package only shortly before the insertion into the human body.

14. Combination package
comprising at least one sterilely packaged implant, especially preferably a dental implant, said implant possibly having undergone an antecedent mechanically and/or chemically abrasive surface modification, and/or optionally having been treated in an aqueous or organic solution of alkali-hydroxides according to one of claims 1-9, and
comprising at least one container with a sterilely packaged aqueous or organic solution of alkali-hydroxides or a mixture of these hydroxides, said solution possibly having been degassed and optionally stored in inert gas, with a total concentration of alkali-hydroxide in the range of 0.005-0.1M.

15. Combination package according to claim 14, wherein the container contains a solution of sodium hydroxide at a concentration of 0.008-0.1 M, preferably of 0.01-0.1 M, especially preferably of 0.01-0.07 M, wherein this solution possibly has been rinsed with an inert gas prior to the bottling for the removal of CO₂ from the solution.

16. Metallic implant with a hydrophilic surface for the at least partial insertion into hard tissue such as into a bone and/or into soft tissue, wherein the hydrophilic metal oxide surface is at least section-wise modified in an alkaline manner, preferably in a weakly alkaline manner, producible or produced by a method according to one of claims 1-13, wherein preferably the implant, at least at its surface or preferably entirely, consists of metal oxide and/or metal and/or wherein preferably the implant contains titanium and/or titanium oxide, which possibly is alloyed with aluminium and niobium, and/or that this alloy contains vanadium instead of niobium,
and/or wherein preferably the implant is a dental implant, of which the surface, which in the implanted state is exposed to the bone and/or soft tissue, is hydrophilized in an alkaline manner,
and/or wherein the hydrophilic surface is preferably at least area-wise macro-rough, preferably modified by sand-blasting and micro-rough, preferably etched by acid.

## Revendications

1. Procédé de fabrication d'un implant métallique ayant une surface hydrophile, destiné à être placé au moins partiellement dans un tissu dur tel qu'un os et/ou dans un tissu mou, **caractérisé en ce que** ledit procédé comprend au moins une étape consistant à traiter au moins une zone destinée à être placée partiellement dans un tissu dur tel qu'un os et/ou dans un tissu mou, le cas échéant suite à une modification de surface préalablement réalisée par enlèvement mécanique et/ou chimique de matière, dans une solution aqueuse ou organique d'hydroxydes alcalins, ou d'un mélange de tels hydroxydes, la concentration totale d'hydroxyde alcalin étant comprise entre 0,005 M et 0,1 M, durant un laps de temps compris entre 2 s et 10 min, ladite étape de traitement à l'hydroxyde alcalin impliquant la modification d'oxydes métalliques en surface, et ledit traitement étant réalisé dans une solution aqueuse d'hydroxydes alcalins, ou d'un mélange de tels hydroxydes, à une température comprise entre 10 et 30°.

2. Procédé selon la revendication 1, **caractérisé en ce qu'**il s'agit d'une solution aqueuse ou organique d'un ou de plusieurs hydroxydes alcalins, la concentration totale mise en oeuvre étant comprise entre 0,05 M et 0,1 M.

3. Procédé selon la revendication 1 ou 2, **caractérisé en ce que** la solution basique est une solution aqueuse constituée exclusivement d'un ou de plusieurs desdites hydroxydes.

4. Procédé selon l'une des revendications précédentes, **caractérisé en ce que** ledit traitement de courte durée est réalisé dans une solution aqueuse d'hydroxydes alcalins, ou d'un mélange de tels hydroxydes, la concentration totale d'hydroxyde alcalin étant comprise entre 0,05 M et 0,1 M.

5. Procédé selon l'une des revendications précédentes, **caractérisé en ce que** ledit traitement est réalisé durant un laps de temps compris entre 5 s et 120 s, de préférence entre 5 et 30 s.

6. Procédé selon l'une des revendications précédentes, **caractérisé en ce que** ladite solution aqueuse est une solution d'hydroxyde de sodium, de préférence une solution d'hydroxyde de sodium d'une concentration de 0,008 à 0,4 M, de préférence de 0,01 M à 0,1 M, avec une préférence particulière de 0,01 M à 0,07 M, la température de travail étant préférentiellement la température ambiante.

7. Procédé selon l'une des revendications précédentes, **caractérisé en ce que**, suite au traitement avec ladite solution aqueuse, la surface de l'implant est stockée et/ou conditionnée sans traitement ultérieur à température élevée et/ou traitement ultérieur de rinçage.

8. Procédé selon l'une des revendications précédentes, **caractérisé en ce qu'**avant son utilisation en tant que bain d'immersion pour l'implant, ladite solution aqueuse est dégazée et, le cas échéant, stockée sous une atmosphère protectrice jusqu'à l'utilisation.

9. Procédé selon l'une des revendications précédentes, **caractérisé en ce que**, durant ledit traitement, la solution est au moins partiellement exposée à des ultra-sons et/ou l'implant est soumis à une lumière UV avant et/ou durant et/ou après le traitement dans la solution.

10. Procédé selon l'une des revendications précédentes, **caractérisé en ce que**, suite au traitement avec ladite solution aqueuse, la surface de l'implant est stockée et conditionnée dans une solution basique, ladite solution basique étant de préférence une solution renfermant de préférence exclusivement des hydroxydes alcalins, ou un mélange de telles solutions, notamment une solution d'hydroxyde de sodium dont la concentration est préférentiellement de 0,0001 à 0,9 M.

11. Procédé selon l'une des revendications précédentes, **caractérisé en ce que** l'on procède, avant le traitement dans ladite solution aqueuse, à une modification de surface par enlèvement mécanique de matière, réalisée sous forme d'un traitement de grenaillage, notamment par sablage, en utilisant de préférence des particules d'oxyde d'aluminium d'une granulométrie moyenne de 0,05 à 0,25 mm ou de 0,25 à 0,5 mm, avec une préférence particulière sous une pression entre 1 et 10 bar, de préférence entre 1 et 6 bar, avec une préférence particulière entre 2 et 5 bar.

12. Procédé selon l'une des revendications précédentes, **caractérisé en ce que** l'on procède, avant le traitement dans ladite solution aqueuse et, le cas échéant, après une modification de surface par enlèvement mécanique de matière selon la revendication 11, à une modification chimique de surface, réalisée notamment par un traitement avec de l'acide sulfurique et/ou de l'acide chlorhydrique et/ou de l'acide fluorhydrique et/ou de l'acide nitrique, de préférence concentrés, ou leurs mélanges, à une température préférentiellement supérieure à la température ambiante.

13. Procédé selon l'une des revendications précédentes, **caractérisé en ce que** l'implant est mis sous conditionnement stérile, le cas échéant suite à une modification de surface préalablement réalisée par enlèvement mécanique et/ou chimique de matière et, optionnellement, après avoir déjà été traité dans une solution aqueuse ou organique d'hydroxydes alcalins, et il est conditionné dans un préemballage combiné le regroupant avec un récipient contenant une solution aqueuse ou organique d'hydroxydes alcalins, ou d'un mélange de tels hydroxydes, dont la concentration globale d'hydroxyde alcalin est comprise entre 0,005 M et 0,1 M, et l'implant n'est retiré dudit conditionnement pour ensuite subir dans ledit récipient un traitement selon l'une des revendications précédentes que peu de temps avant son placement dans le corps humain.

14. Préemballage combiné
contenant au moins un implant sous conditionnement stérile, s'agissant préférentiellement d'un implant dentaire, ledit implant ayant le cas échéant subi une modification de surface réalisée préalablement par enlèvement mécanique et/ou chimique de matière, et/ou ayant été traité optionnellement dans une solution aqueuse ou organique d'hydroxydes selon l'une des revendications 1 à 9, et
contenant en outre au moins un récipient comportant une solution aqueuse ou organique d'hydroxydes alcalins, ou d'un mélange de tels hydroxydes, mise sous conditionnement stérile, le cas échéant dégazée et optionnellement mise sous atmosphère protectrice, sa concentration totale d'hydroxyde alcalin étant comprise entre 0,005 M et 0,1 M.

15. Préemballage combiné selon la revendication 14, **caractérisé en ce que** ledit récipient contient une solution d'hydroxyde de sodium dont la concentration est de 0,008 à 0,1 Mol, de préférence de 0,01 à 0,1 Mol, avec une préférence particulière de 0,01 à 0,07 Mol, ladite solution ayant le cas échéant été soumise à un flux d'un gaz inerte avant être conditionnée pour ainsi chasser le CO₂ contenu dans la solution.

16. Implant métallique ayant une surface hydrophile, destiné à être placé au moins partiellement dans un tissu dur tel qu'un os et/ou dans un tissu mou, **caractérisé en ce que** ladite surface en oxyde métallique hydrophile a subi, au moins dans certaines zones, une modification la rendant basique, de préférence faiblement basique, pouvant être fabriqué ou ayant été fabriqué selon un procédé selon l'une des revendications 1 à 13, celui-ci étant constitué préférentiellement, au moins à sa surface et de préférence entièrement, d'oxyde métallique et/ou de métal et/ou que celui-ci contient préférentiellement du titane et/ou de l'oxyde de titane, ce dernier formant le cas échéant un alliage avec un complément en aluminium et en niobium, et/ou que ledit alliage contient de préférence du vanadium au lieu du niobium, et/ou qu'il s'agit préférentiellement d'un implant dentaire dont la surface laquelle est exposée, suite à la pose de l'implant, à l'os et/ou à un tissu mou a été soumise à une hydrophilisation basique et/ou que ladite surface hydrophile présente de préférence, au moins dans certaines zones, une macro-rugosité, s'agissant notamment d'une modification par sablage, et une micro-rugosité, obtenue notamment par gravure acide.
